# EUROPEAN PATENT SPECIFICATION

(11) **EP 3 804 793 B1**
(45) Date of publication and mention of the grant of the patent: **07.05.2025**
(21) Application number: 20209315.9
(22) Date of filing: 30.09.2016
(51) Int. Cl.: A61M 11/00, A61M 16/00, A61M 16/12

(54) **DEVICE FOR DIFFUSING HIGH CONCENTRATION NO WITH INHALATION THERAPY GAS**
GERÄT ZUR DIFFUSION VON HOCHKONZENTRIERTEM NO MIT INHALATIONSTHERAPIEGAS
DISPOSITIF DE DIFFUSION D'UN GAZ THÉRAPEUTIQUE PAR INHALATION À HAUTE CONCENTRATION NO

(30) Priority: 01.10.2015 US 201562235798 P
(43) Date of publication of application: 14.04.2021
(62) Divisional of application: 16778660.7
(73) Proprietor: Mallinckrodt Hospital Products IP Limited, Dublin (IE)
(72) Inventor: FALLIGANT, John C., Mount Horeb, Wisconsin 53572 (US); TOLMIE, Craig R., Stoughton, Wisconsin 53589 (US)
(74) Representative: FRKelly

(56) References cited:
- CA-A1- 2 874 999
- US-A- 5 522 381
- US-A- 5 871 009
- US-A1- 2001 037 810

## Description

### TECHNICAL FIELD

Principles and embodiments of the present invention relate generally to a device for combining nitric oxide (NO) with other gases being administered to a patient for inhalation therapy.

### BACKGROUND

A number of gases have been shown to have pharmaceutical action in humans and animals. One such gas is nitric oxide (NO) that, when inhaled, acts to dilate blood vessels in the lungs, improving oxygenation of the blood and reducing pulmonary hypertension. In the field of inhalation therapy for various pulmonary conditions such as acute pulmonary vasoconstriction, hypertension and thromboembolism, or inhalation injury, treatment has included the use of the therapeutic gas NO supplied from a gas cylinder. More specifically, this gaseous NO for inhalation therapy is supplied to a patient from a high pressure gas cylinder containing NO. For example, such an approach is disclosed in U.S. Patent No.: 5,558,083 entitled "Nitric Oxide Delivery System", which is incorporated herein by reference in its entirety.

Inhaled nitric oxide (INO) therapy, generally speaking, involves delivering a concentration of NO, at a set dose, to mechanically ventilated patients. NO delivery systems of this type (wrap-around style) can sense fresh gas flow in the inspiratory limb of the mechanical ventilator, and ratio-metrically deliver NO from source cylinders into the inspiratory limb of the ventilator, via an injector module, to achieve a prescribed patient dose.

Typically speaking, the concentration of the NO source (e.g., from the source cylinders) may be about 800 ppm NO. As discussed above, this NO source gas at 800 ppm can be proportionally delivered (e.g., ratio-metrically delivered) into fresh gas flow such that the concentration of NO in the fresh gas flow is about 5 to 80 ppm.

Although INO therapy has many benefits, it has been found that when delivering NO into fresh gas flow, nitrogen dioxide (NO₂), a toxic gas, can be generated by reacting with O₂ in fresh gas flow. More specifically, the formation of nitrogen dioxide is proportional to the square of the NO concentration multiplied by the concentration of O₂.

The kinetics and rate equation for the conversion of NO to NO₂ is given by:

2 NO → N₂O₂

N₂O₂ + O₂ → 2 NO₂

Thus, giving a formation rate of NO₂ = *k*[NO]²[O₂], where k is in units of L•mol⁻¹•s⁻¹, or in partial pressures for the gases.

Accordingly, the amount of NO₂ produced (ppm NO₂) is related to the square of the NO concentration and is linear to the oxygenation concentration and time.

In light of the above, NO delivered into a ventilator breathing circuit from a low concentration NO source (*e.g**.,*** 100 ppm, 400 ppm, and 800 ppm NO cylinders) may not result in undesirably high amounts of NO₂, for example > 1 ppm; however, following the above kinetics, the use of NO delivered into a ventilator breathing circuit from a high concentration source *(e.g.,* 2000 ppm, 5000 ppm, and 10,000 ppm NO cylinders) would be expected to generate an unacceptable amount of toxic NO₂, for example > 1 ppm NO₂ generated when providing a 40 ppm NO dose with 60% O₂. Theoretically, for the same NO therapy dose, NO₂ from a 5000 ppm source gas may have a formation rate to 39 times greater than an 800 ppm source.

Some have attempted to address this problem using varying techniques; however, these techniques may not work in specific systems, may not work when delivering high concentration NO, may not work at all, or can fail to address the actual cause of NO₂ generation and/or underlying factors in NO₂ generation not previously appreciated. Accordingly a need exists for systems and methods of reducing NO₂ generation that work in specific systems, address the actual cause of NO₂ generation and/or the underlying factors not previously appreciated.

US5871009A discloses that, during the administration of NO to a patient, NO reacts with oxygen in the respiratory gas to form NO2. Since NO2 is a toxic gas, minimizing the formation of this gas is important. A previously unknown property of NO is that an initial amount of NO2 is formed when NO and O₂ mix. By adding NO to respiratory gas at a number of spaced-apart mixing points, the total initial amount of NO2 formed can be reduced. In a method and a device for mixing and administering NO, the gas containing NO is supplied to a flow divider for distributing the flow and sending sub-flows to a number of various mixing points.

Patent document CA2874999A1 is hereby acknowledged .

### SUMMARY

The invention is defined by the appended claims.

The present disclosure provides a device for use in combining a first gas stream comprising molecular oxygen (O2) and a second gas stream comprising nitric oxide (NO) for delivery of a combined gas stream to a patient as detailed in claim 1. Advantageous features are provided in dependent claims.

Systems and methods of the present invention can be used to reduce NO₂ generated when, for example, being delivered into fresh gas flow in a ventilator breathing circuit. Further, systems and methods of the present invention can enable high concentration NO to be delivered into ventilator breathing circuits, via a diffusing device, without generating undesirably large amounts of NO₂ for example > 1 ppm NO₂ for a dose of 40 ppm NO with 100% O₂. Use of high concentration NO sources *(e.g.,* 2000, 4880, 10,000 ppm NO cylinders) can provide benefits such as, but not limited to, the use of smaller NO gas cylinders, which allows increased portability and introducing smaller volumes of the high concentration gas into the ventilator gas stream, and less dilution of oxygen-enriched Fresh Gas Flow (FGF) by the NO and carrier N₂ gases. It has surprisingly been found that introduction of smaller NO volumes with diffusion at equivalent or higher rates can generate less NO₂ overall with shorter diffusion time associated with smaller gas volume. The issues addressed herein relate to at least rapidly reducing NO concentration before large concentrations of NO₂ can be formed.

### BRIEF DESCRIPTION OF THE DRAWINGS

Further features of embodiments of the present disclosure, their nature and various advantages will become more apparent upon consideration of the following detailed description, taken in conjunction with the accompanying drawings, which are also illustrative of the best mode contemplated by the applicants, and in which like reference characters refer to like parts throughout, where:
FIGS. 1A-F show the NO₂ generated after injecting NO into oxygen-enriched air under various conditions;
FIG. 1G shows the NO₂ generated at various points downstream from the point of NO injection;
FIGS. 2A-C illustrate an exemplary embodiment of a mixing device having a plurality of blades;
FIGS. 3A-C illustrate an exemplary embodiment of a tapered section having a plurality of angled fins;
FIGS. 4A-C illustrate an exemplary embodiment of a mixing device having a plurality of plates;
FIGS. 5A-C illustrate an exemplary embodiment of a mixing device having a plurality of curved blades;
FIGS. 6A-C illustrate an exemplary embodiment of a mixing device having a plurality of curved blades and an injection channel at a tapered section;
FIG. 7 illustrates an exemplary velocity distribution of a gas flow within a tube;
FIGS. 8A-B illustrate an exemplary embodiment of a device for diffusing a high NO source concentration, low volume gas flow and a high volume gas flow;
FIGS. 8C-D illustrate another exemplary embodiment of a device for diffusing a high NO source concentration, low volume gas flow and a high volume gas flow;
FIG. 9A illustrates an exemplary embodiment of a tapered section having a funnel shape;
FIG. 9B illustrates an exemplary embodiment of a tapered section having a cone shape;
FIG. 9C illustrates an exemplary embodiment of a tapered section having a bell shape;
FIG. 10 illustrates an exemplary embodiment of a bi-directional tapered section;
FIG. 11 illustrates an exemplary tapered section depicting a convex contour of an inside surface of a tapered section wall;
FIG. 12 illustrates an exemplary embodiment of a second gas passing through an injection channel into a first gas passing through a tapered section;
FIG. 13 illustrates an exemplary embodiment of a diffusing device inserted into a ventilator circuit;
FIG. 14 shows a comparison of the NO₂ generated during mechanical ventilation using an exemplary diffuser described herein and a conventional low source concentration injector module;
FIGS. 15A-F show a comparison of the NO₂ generated under steady state FGF flow conditions within smooth bore tubing using an exemplary diffuser described herein, an exemplary accelerator as described herein, and a conventional low source concentration injector module;
FIG. 16 shows the reduction of the NO₂ generated by heating an exemplary ventilator breathing circuit;
FIG. 17 shows the NO₂ generated in the initial region with various NO source cylinder concentrations ranging from 800 ppm to 9760 ppm with a gas velocity ratio (FGF : NO) of approximately 1:1;
FIGS. 18A-D show the NO₂ generated in the initial region with various NO source cylinder concentrations ranging from 800 ppm to 9760 ppm with a varying gas velocity ratio (FGF:NO) and a set dose of 10 ppm NO;
FIG. 19 shows the NO₂ generated in the initial region with a 4880 ppm NO source cylinder concentration and a set dose of 40 ppm, with a varying gas velocity ratio (FGF:NO);
FIGS. 20A-B show the NO₂ generated in the initial region with various NO source cylinder concentrations ranging from 800 ppm to 9760 ppm with a varying gas velocity ratio (FGF:NO) and a set dose of 10 ppm NO;
FIG. 21 shows the NO₂ generated in the initial region with a 4880 ppm NO source cylinder concentration and a set dose of 40 ppm, with a varying gas velocity ratio (FGF:NO); and NO₂ generated during homogeneous phase of 40 ppm equal to set dose; and
FIGS. 22A-B show the NO₂ generated in ppm and as a percentage of the set dose with simulation of expiratory time period change relative to inspiratory time period with higher flows.

### DETAILED DESCRIPTION

The present technology, generally speaking, is directed to systems and methods of injecting NO into fresh gas flow in the inspiratory limb of a ventilator breathing circuit such that NO₂ generation is minimized. The present technology takes advantage of previously unknown factors which applicant surprisingly found affect NO₂ generation. More specifically, systems and methods of the present technology can be used to deliver NO from a source of high concentration NO (*e.g.,* 5000 ppm NO source) into fresh gas flow in the inspiratory limb of a ventilator breathing circuit such that NO₂ generation is substantially minimized and/or the NO₂ generated is within a desired range (*e.g.,* less than 1 ppm NO₂ delivered to the patient, the same or less NO₂ as generated by substantially lower concentration NO sources using conventional injector modules, etc.) by factoring in variables such as, but not limited to, location of injection of NO into fresh gas flow, fresh gas flow velocity, NO flow velocity, and/or ratio of impinging velocity of NO and fresh gas flow, to name a few.

Further, systems and methods of the present technology can be used with a ventilator breathing circuit by not substantially causing pressure drop, for example less than 1.5 cm H₂O at 60 SLPM, minimizing flow profile changes, minimizing the increase in the compressible volume of fresh gas flow, and/or enabling for patient spontaneous breathing in the ventilator breathing circuit. Further still, systems and methods of the present technology can be used immediately downstream from flow sensors that require the fresh gas flow be laminar and/or can be used immediately upstream from at least one gas sampling line.

"Compressible volume" means the volume of a conduit and all components in fluid communication with and in line with the flow path of the conduit. For example, the compressible volume of breathing circuit is the volume of the breathing circuit and all of the components within it (*e.g*. humidifier, injector module, sample T's).

As used herein, "diffusion", "diffusing" and related terms refer to the overall transport of molecules of one gas *(e.g.* NO) into and throughout a stream of another gas (*e.g.* oxygen-enriched air). The use of the terms "diffusion", "diffusing" and related terms does not exclude the contribution of bulk fluid motion or other transport phenomena to the mixing and homogenization of two or more gas streams.

As noted above, prior to applicant's research, it was believed that NO₂ formation was predicated on the concentration of NO and O₂ (*e.g.,* parts-per-million of NO, percent of O₂ (otherwise known as FiO₂)), as well as the distance/dwell time between gas mixing and the patient. Following this belief, delivery of NO from a high concentration source (*e.g.,* 5000 ppm, 10,000 ppm NO cylinder) would result in substantially high levels of NO₂. For example, a 4880 ppm NO cylinder concentration reduced down to a set dose of 10 ppm is a turn down ratio of 488:1, whereas a 800 ppm cylinder concentration reduced down to a 10 ppm set dose has a turn down ratio of 80:1, theoretically NO₂ is generated at a rate approximately 37 times greater with a 4880 ppm NO supply than with a 800 ppm NO supply cylinder for a dose of 10 ppm. Without a means of overcoming this problem, high concentrations sources of NO cannot be used for INO therapy as this would result in delivery of undesirably high levels of NO₂ to a patient, and many benefits associated with using high concentration sources of NO for INO therapy (e.g., smaller NO supply cylinders, increased portability of INO therapy devices, smaller volumes of NO-containing gas (e.g., nitrogen and NO gas blends) in the breathing circuit, reduced inspired oxygen dilution due to smaller injected NO-containing gas volumes, etc.) would be unrealized.

In exemplary embodiments, using a higher concentration source gas can reduce a portion of the NO₂ delivered to a patient. For example, the higher the NO concentration of the source gas, the smaller the volume of source gas required to be delivered to obtain the desired set NO dose. Even with the same NO₂ concentration in the source gas (*e.g.* the same NO₂ concentration in a gas cylinder), by using this lower volume of source gas, less volume of NO₂ from the source gas would be delivered and hence the patient receives less NO₂ from the NO source (*e.g.* cylinder).

In light of at least these unrealized benefits, applicant conducted extensive research and testing into NO₂ generation when injecting NO into the inspiratory limb of a ventilator breathing circuit.

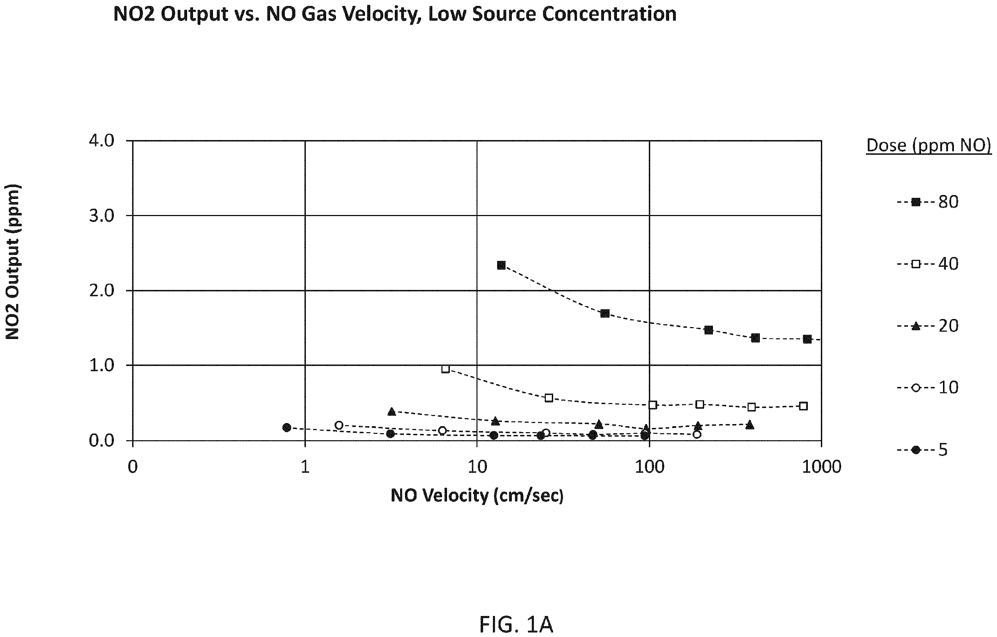

From this research and testing, it was surprisingly found that NO₂ formation was greater during the expiratory phase of ventilation, in which fresh gas flow in the inspiratory limb of a ventilator is substantially slower, laminar (non-turbulent) than during the inspiratory non-laminar (turbulent) phase of ventilation. With this knowledge, further research and testing was conducted to determine the relationship between the NO₂ output and variables such as the impinging velocity of the NO-containing gas, the flow rate of the FGF, and the NO dose. In each of these experiments, the fresh gas was oxygen-enriched (e.g. 60% O₂/air), the NO₂ concentration was measured at a distance beyond the NO injection point *(e.g.* 1,000 mm), and the NO source concentration was either a low concentration (e.g. 800 ppm NO) or a high concentration (e.g. 4880 ppm NO). The NO was injected and the gases were mixed using a conventional injector module. The results of this testing are shown in Tables 1-2 and FIGS. 1A-F.

**Table 1 - NO₂ Generated with Low Concentration NO Source**

| | | NO₂ Delivery (ppm NO₂) | | | | |
|---|---|---|---|---|---|---|
| | | NO Dose | | | | |
| | | 5 | 10 | 20 | 40 | 80 |
| FGF Flow Rate (SLPM) | 0.5 | 0.17 | 0.2 | 0.39 | 0.95 | 2.3 |
| | 2 | 0.089 | 0.13 | 0.26 | 0.56 | 1.7 |
| | 8 | 0.067 | 0.101 | 0.22 | 0.47 | 1.5 |
| | 15 | 0.065 | 0.08 | 0.16 | 0.48 | 1.4 |
| | 30 | 0.065 | 0.096 | 0.2 | 0.44 | 1.4 |
| | 60 | 0.063 | 0.081 | 0.21 | 0.46 | 1.3 |

**Table 2- NO₂ Generated with High Concentration NO Source**

| | | NO₂ Delivery (ppm NO₂) | | | | |
|---|---|---|---|---|---|---|
| | | NO Dose | | | | |
| | | 5 | 10 | 20 | 40 | 80 |
| FGF Flow Rate (SLPM) | 0.5 | 1.9 | 2.6 | 3.4 | 2.9 | 3.3 |
| | 2 | 0.8 | 0.63 | 0.36 | 0.57 | 1.7 |
| | 8 | 0.088 | 0.089 | 0.14 | 0.41 | 1.2 |
| | 15 | 0.073 | 0.075 | 0.11 | 0.34 | 1.2 |
| | 30 | 0.047 | 0.089 | 0.13 | 0.34 | 1.2 |
| | 60 | 0.032 | 0.055 | 0.14 | 0.34 | 1.2 |

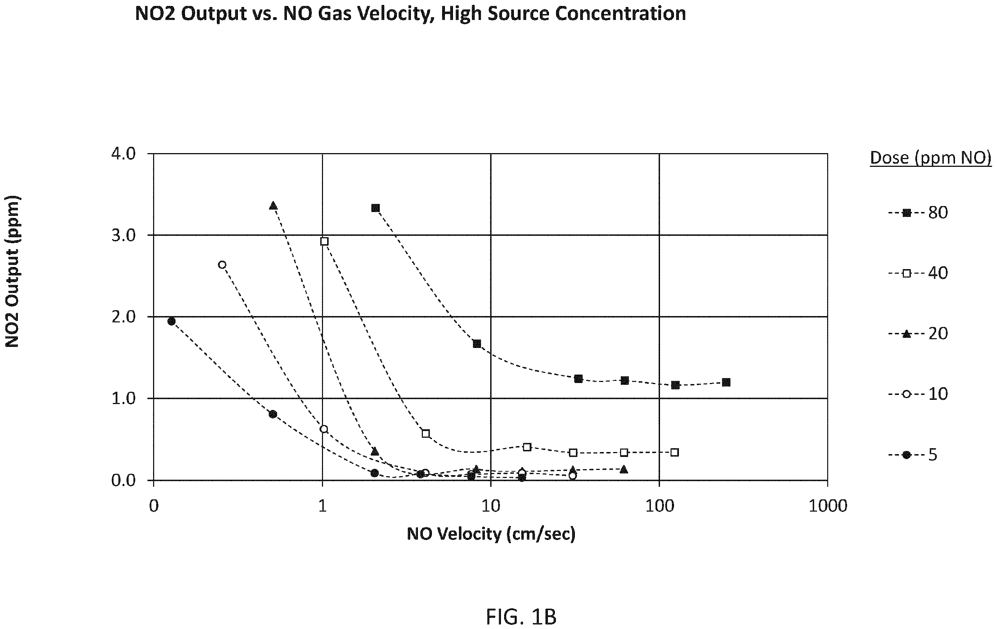

FIGS. 1A and 1B show that the impinging velocity of NO with fresh gas flow in the breathing circuit can substantially impact the amount of NO₂ generated. Also, as can be seen by comparing FIG. 1A (low concentration) and FIG. 1B (high concentration), increasing the NO concentration generally increased the amount of NO₂ produced.

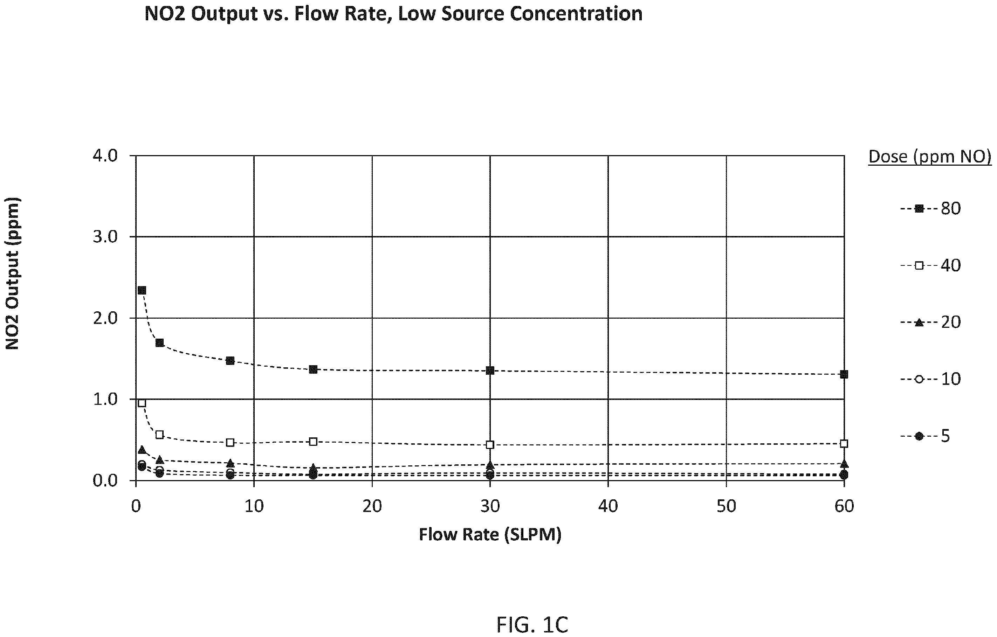

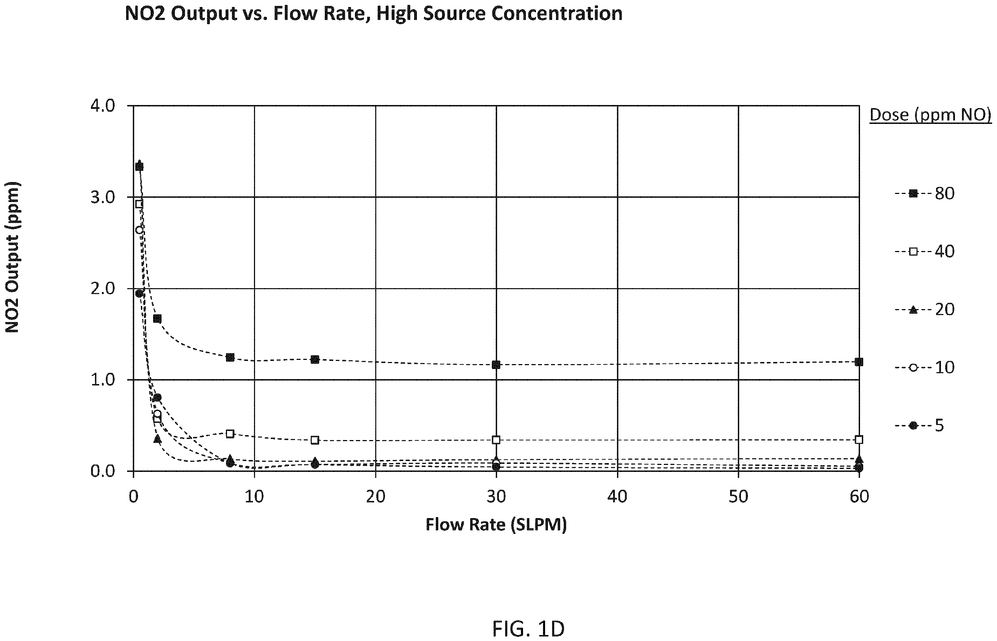

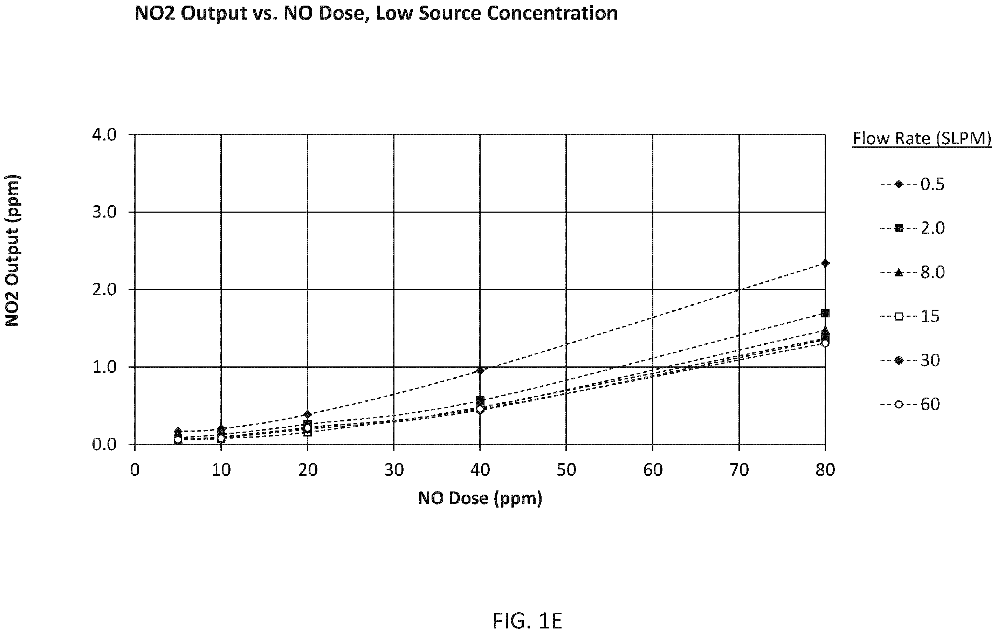

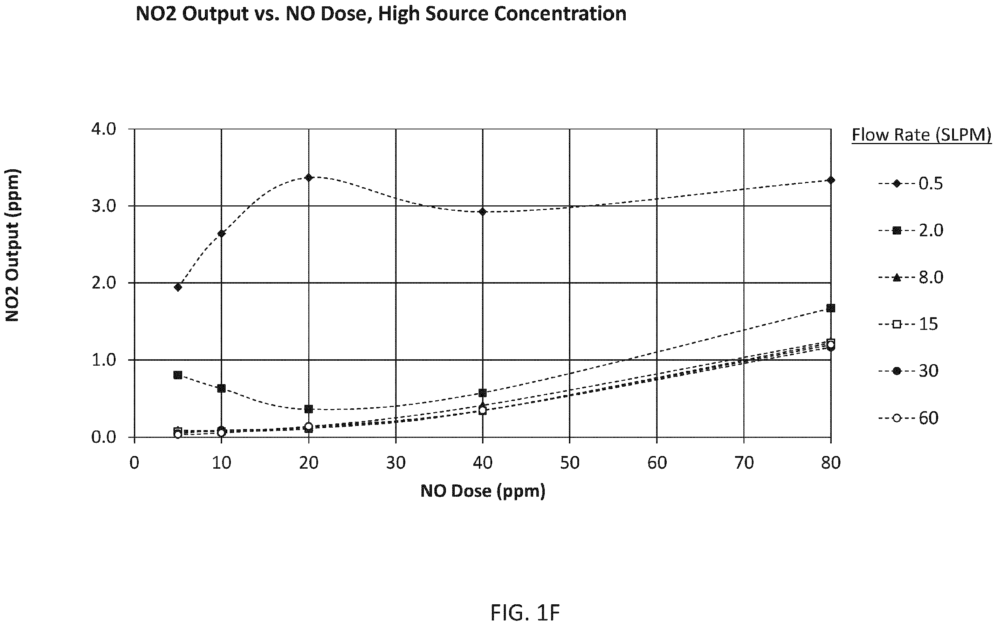

FIGS. 1C-1F show the respective amounts of NO₂ generated for different set NO dosages when the NO is injected into the FGF having different flow rates. As can be seen by comparing FIG. 1C (low concentration) and FIG. 1D (high concentration), increasing the NO concentration generally increased the amount of NO₂ produced, particularly at the lower flow rates of FGF. This is also shown by comparing FIG. 1E (low concentration) and FIG. 1F (high concentration), as the NO₂ output curve for 0.5 SLPM was drastically different between the low NO source concentration and the high NO source concentration.

Although the above is beneficial in understanding NO₂ generation, it substantially complicates minimizing NO₂ generation when NO *(e.g.,* from a high concentration NO source) is being injected into the inspiratory limb of the ventilator breathing circuit. For example, the fresh gas flow velocity can vary (*e.g.,* the fresh gas flow rate can vary over the course of the patient breathing cycle, etc.); the NO velocity injected into the fresh gas flow can vary (*e.g.,* the NO flow rate can vary depending on the pressure in the NO delivery line, the dimensions of the NO injection port at the diffusing device, the dimensions of the NO delivery conduit in the NO delivery system, to name a few); and ratio-metric delivery, as may be required for INO therapy, for example, to achieve a constant inspired NO concentration, can require varying the NO delivered in proportion to the fresh gas flow. During the expiratory phase some ventilators use low bias flows (0.5 SLPM) and have slower FGF in a ventilator breathing circuit, which may generate more NO₂ than during the inspiratory phase (faster FGF in the ventilator breathing circuit). For example, the data above shows that 10 to 20 times more NO₂ may be generated with 4880 ppm NO than with 800 ppm NO at low FGF associated with ventilator exhalation bias flows over the same time period, where insufficient diffusing may occur with a conventional injector module.

Accordingly, in exemplary embodiments, a diffusing device can be designed to minimize NO₂ generation by controlling the impinging velocity of the NO and fresh gas flow and the location of injection of the NO into the FGF. In various embodiments, the velocity of the NO flow stream may be high enough relative to the FGF at the location the NO is injected to minimize the NO₂ generated. Without being bound by theory, it is thought the NO flow stream may penetrate the FGF stream perpendicularly and with proportional velocities. With very low NO velocity relative to FGF velocity, without being bound by theory, it is believed the NO stays at the outer wall of the FGF stream resulting in poor mixing. Conversely, if only the NO velocity is high and the FGF is not, the mixing time can also be extended resulting in high NO₂.

While not wishing to be bound by any particular theory, it is believed that the initial contact diffusion rate of the two mixing gas streams may be primarily controlled by the molecular kinetic energy. In such a gas impingement mixing process, the dissipative exchange from gas momentum can provide direct acting mixing. This rapid diffusion can take place immediately in the vicinity of the nozzle outlet, or directly at the gas impingement point. The molecular kinetic energy is defined as ½ times the molar mass times the square of the velocity, and thus the velocity is inversely proportional to the square root of the molar mass. Equivalent volumes of different gases contain the same number of particles, and the number of moles per liter at a given temperature and pressure is constant. This indicates that the density of gas is directly proportional to its molar mass. Accordingly, this indicates the same mixing energy (i.e. same kinetic energy) would exist at approximately equal velocities or a ratio of 1:1, due to the similar molecular weights of NO, N₂, air and O₂, which all range from 28 to 32 grams per mole. However, given the slight molecular weight imbalance between air/O₂ mixtures and NO/N₂ mixtures, the greatest diffusion from the dissipative energy exchange can be at velocity ratios less than 1:1 (FGF: NO), such as 0.85:1, 0.9:1 or 0.95:1, depending on the relative proportions of N₂, NO, O₂ and air.

In various embodiments, the velocity of the two gas streams may be proportional to each other in order to minimize the NO₂ generated. The NO velocity can be controlled by changing the dimensions of the NO injection port, for example, as other factors (*e.g*., pressure in the NO delivery line, dimensions of the NO injection channel, etc.) may be fixed. It will be appreciated that any means for controlling the NO velocity can be used. However, controlling the fresh gas flow velocity can be substantially challenging as the velocity of the fresh gas flow is typically controlled by the ventilator. Further, as noted above, the velocity of the fresh gas flow during the expiratory phase can be substantially slow. In at least some instances, the impinging velocity of the fresh gas flow during at least the expiratory phase can be too slow to minimize NO₂ generation. Accordingly, in exemplary embodiments, the diffusing device can include at least one accelerator capable of accelerating the fresh gas flow to a desired impinging velocity, for example, that may be directed to a point of intersection with the NO-containing gas.

In one or more embodiments, the orifice diameter at the NO gas impingement point to the fresh gas flow tube can be sized appropriately to maintain a fixed aspect ratio outlet area between the diffusing module 100 tube diameter (i.e. the FGF tube diameter) to the NO nozzle outlet diameter area (i.e. the injection port orifice diameter). This ratio in tube outlet area can be proportional to the NO cylinder concentration over the NO set dose. For example, for an 800 ppm cylinder concentration at a set dose of 20 ppm, a 40 to 1 turn down ratio exists in NO flow rate. In order to maintain a 1:1 impinging gas velocity relationship, an injector module flow tube area to injection nozzle outlet area may be sized at 40:1 at the lowest expected fresh gas flow rate (*e.g.,* 0.5 SLPM). As another example, for a 4880 ppm cylinder concentration at a set dose of 10 ppm, a 488 to 1 turn down ratio exists in NO flow rate. In order to maintain a 1:1 impinging gas velocity relationship, an injector module tube area to injection nozzle outlet area may be sized at 488:1

In one or more embodiments, the dimensions of the injection channel and injection port may be adjusted so the ratio of NO velocity to FGF velocity is less than about 2:1, such as about 1.5:1, 1:1, 0.95:1, 0.9:1, 0.85:1, 0.8:1, 0.7:1, 0.6: 0.5:1, 0.4:1, 0.3:1, 0.2:1, 0.1:1 or 0.05:1.

In exemplary embodiments, at least one accelerator can be any device or component capable of accelerating all or a portion of the fresh gas flow. For example, the accelerator can be a conical structure with a tapered surface, a tapered section, bi-directional conical structure, and/or any shapes or surfaces capable of accelerating fresh gas flow. Other examples include structures with surfaces similar to a wing, as gas flowing over the top of a wing (curved surface) has a faster velocity than the gas flowing underneath the bottom of the wing (relatively flat surface). These accelerator structures are only exemplary, and other structures capable of accelerating at least some portion of a gas flow are also within the scope of this technology.

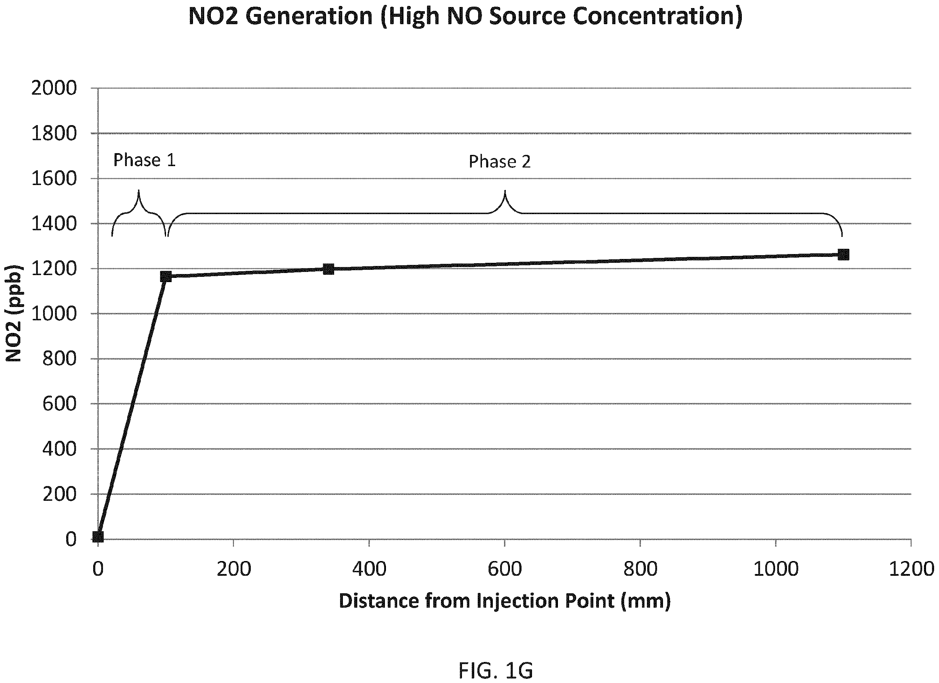

Notably, when injecting NO into fresh gas flow, the device's configuration and dimensions may be adjusted to reduce the source NO concentration as quickly as possible. In various embodiments, mixing features may be added to the device downstream of the NO injection point. In various embodiments, mixing can be thought of in 2 phases. The first phase where the majority of NO₂ may be generated is the time from NO injection to when the NO concentration reaches the set dose (*e.g.,* a homogeneous state equal to the set dose). The second phase of NO₂ generation is due to the residence time in the inspiratory limb at set dose. A majority of NO, may be generated at, or near, the first point of contact between the NO and fresh gas flow (*e.g.,* O₂). These two phases of NO generation can be seen in FIG. 1G, which shows the NO₂ concentration at various points downstream from the point of injection. As can be seen from FIG. 1G, the majority of the NO₂ is generated soon after the NO is injected (Phase 1), with only a small portion of the NO₂ being generated after the initial injection and mixing of the NO (Phase 2).This majority of the NO₂ being generated during the first phase follows the above NO₂ generation kinetics as the first phase of NO injection the local NO concentration is highest (e.g., as the NO has not yet diffused into the fresh gas flow to provide the homogenous set NO dose). By way of example, when injecting 5000 ppm NO into the breathing circuit, at the point of injection the NO concentration is highest (e.g., approximately 5000 ppm NO) as the NO has not yet diffused with the fresh gas flow. After this point of injection, the injected NO and the fresh gas flow diffuse together causing the NO concentration to decrease to a lower concentration (*e.g*., from 5000 ppm NO to a desired dose of 20 ppm NO).

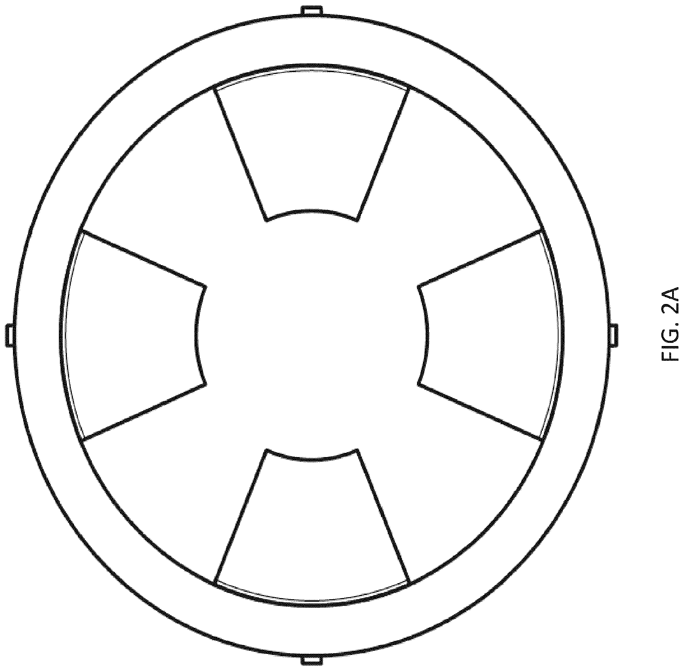

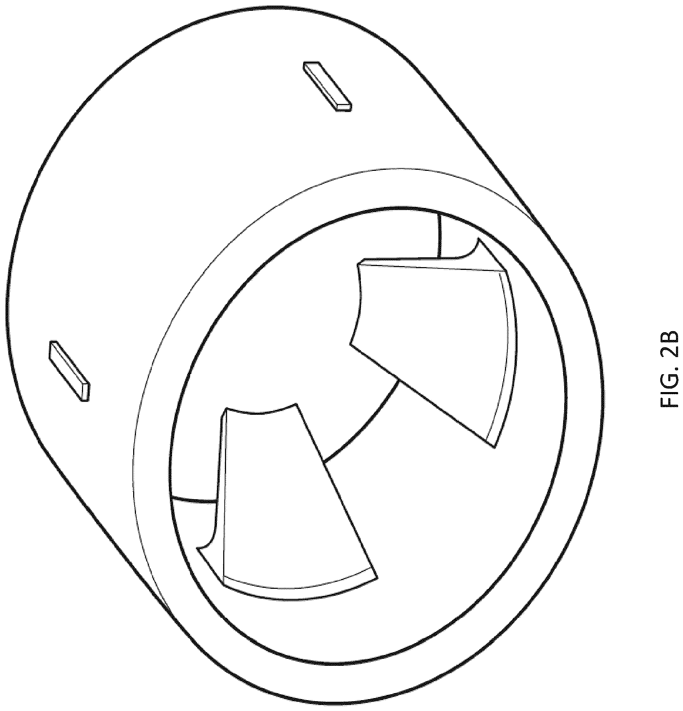

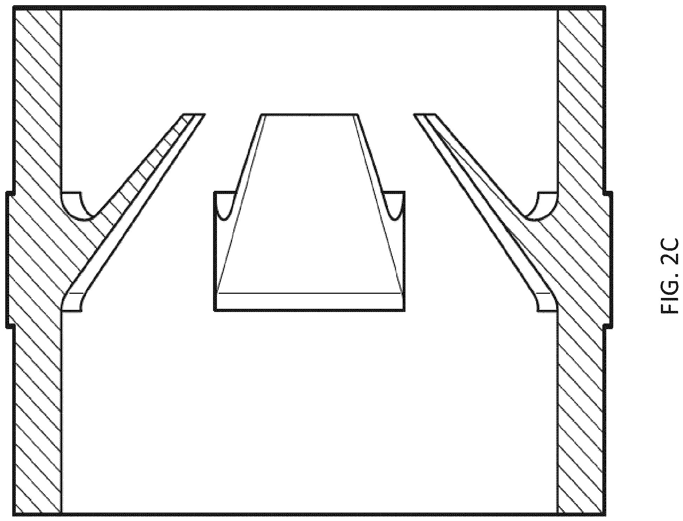

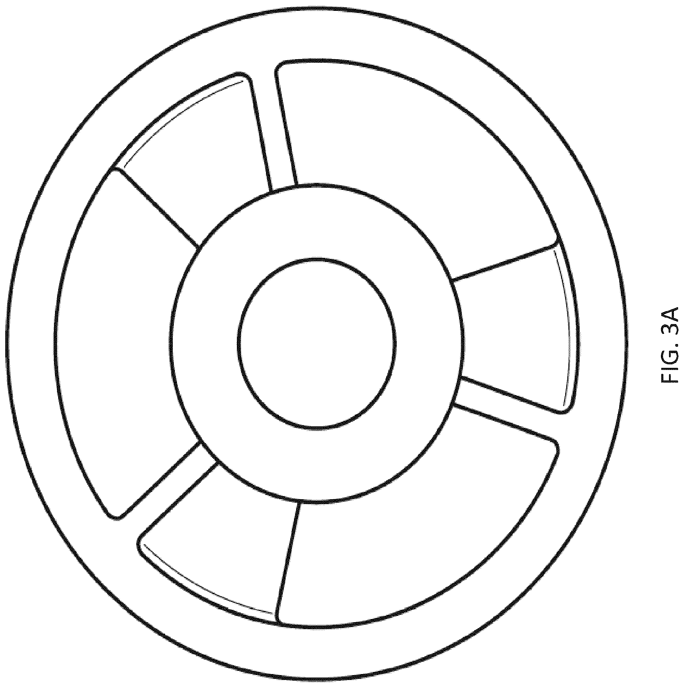

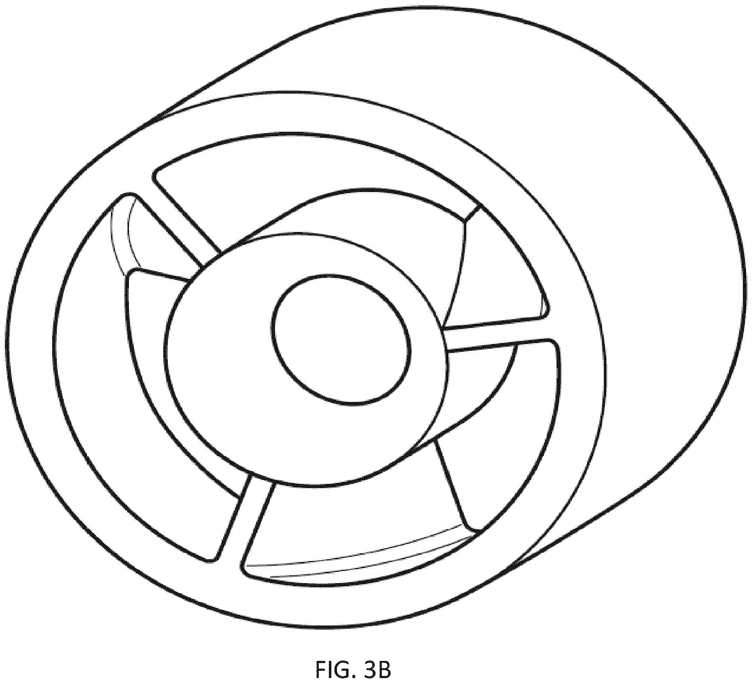

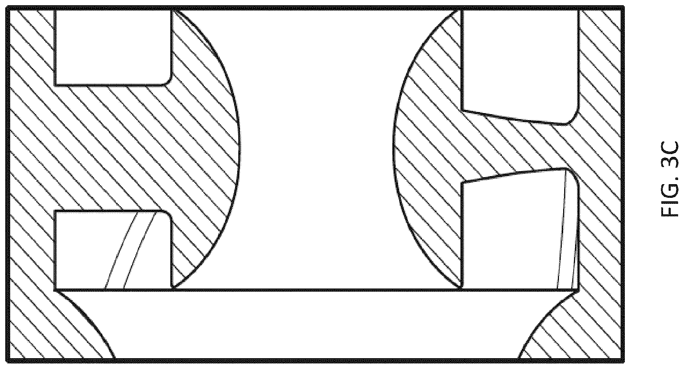

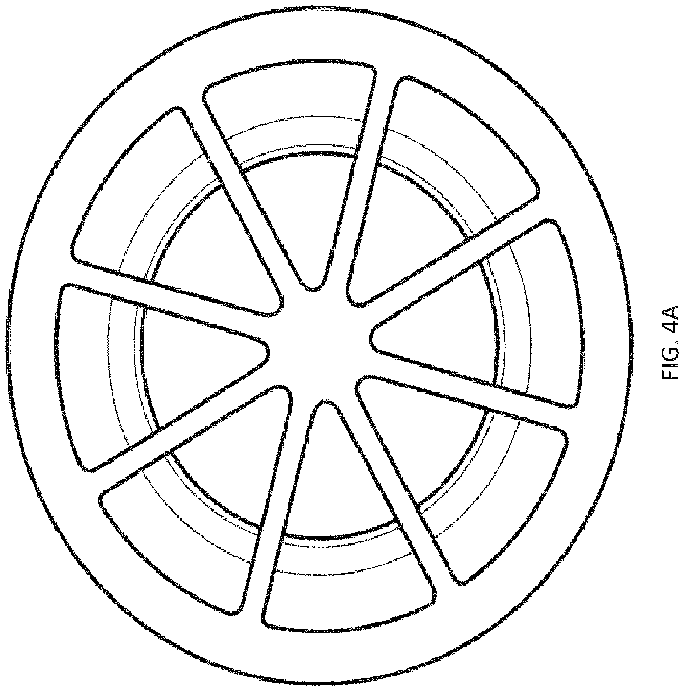

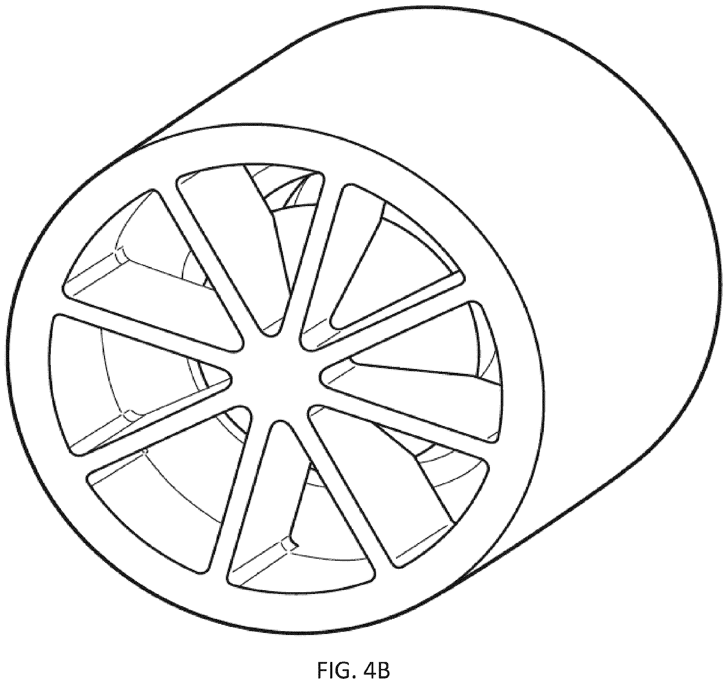

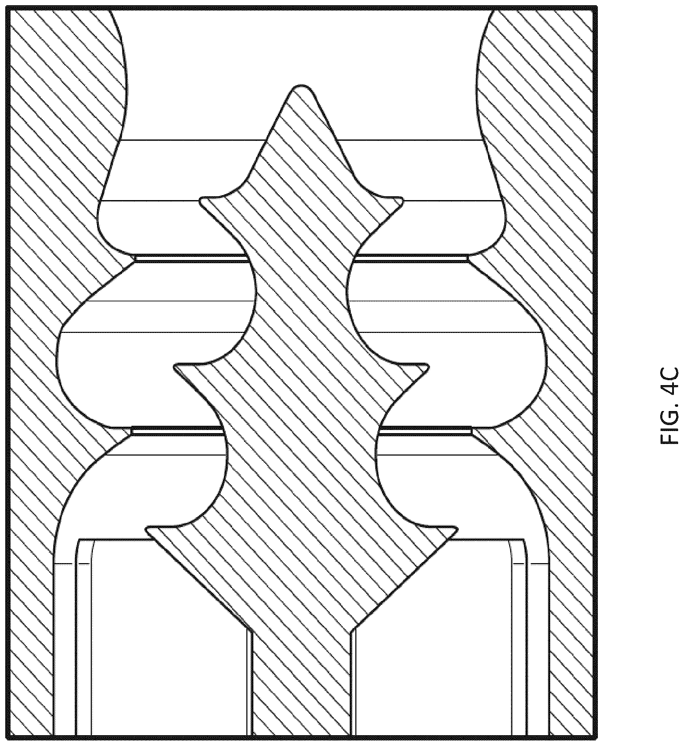

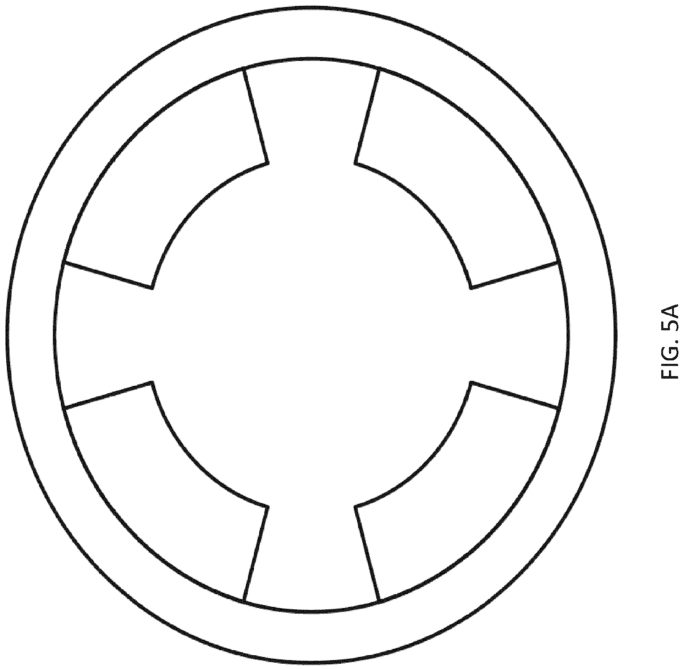

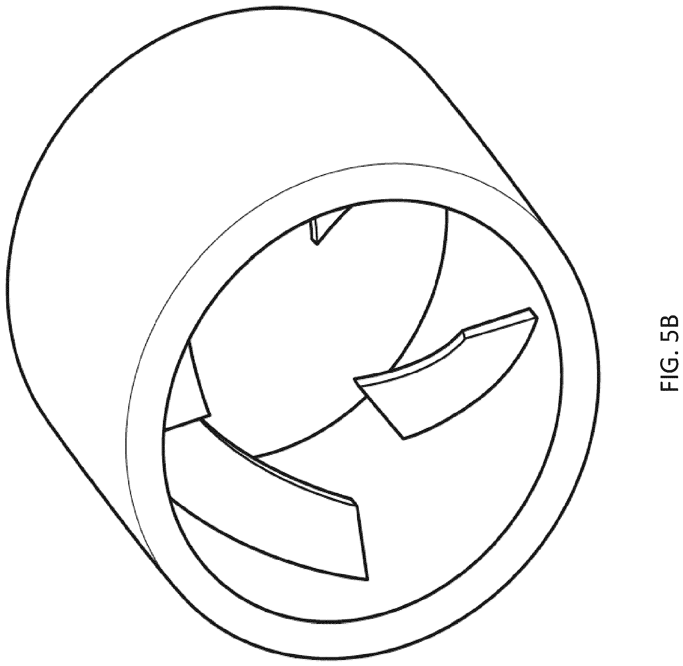

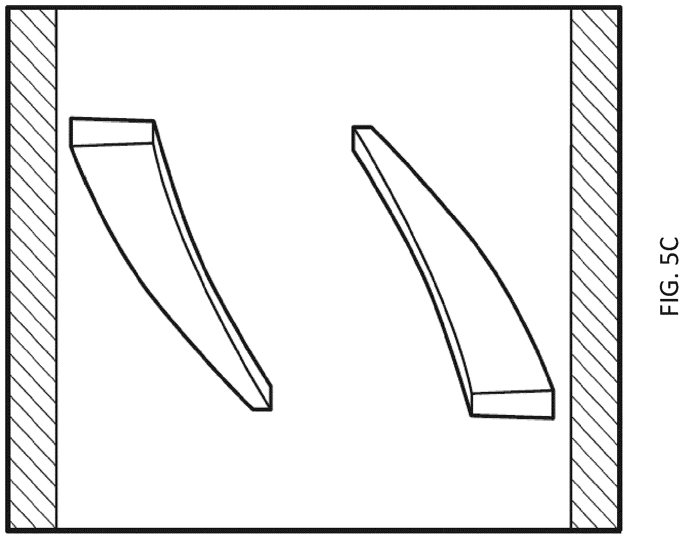

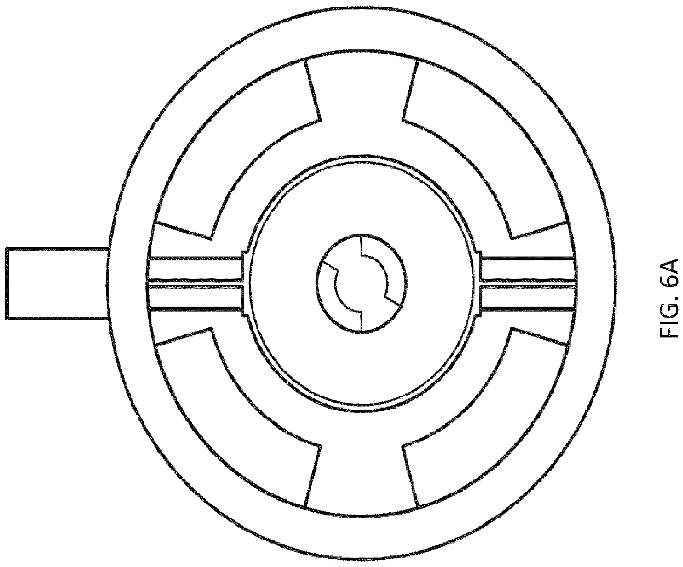

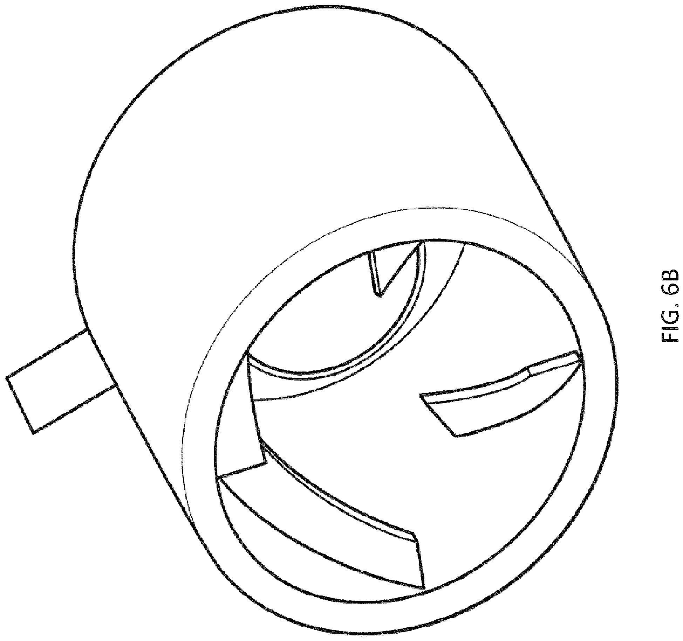

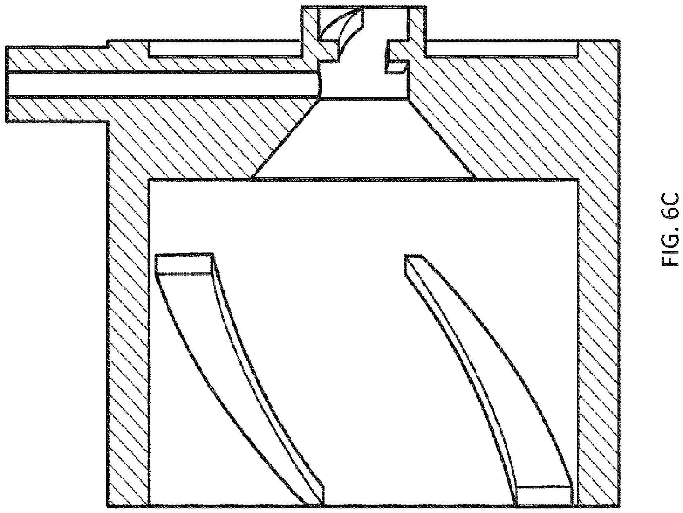

Accordingly, one approach for rapidly mixing the NO and FGF is the use of a mixing device placed immediately downstream or close to the point of NO injection to ensure that the combined gas stream has a homogenous NO concentration as soon as possible. For example, a plurality of blades, plates and/or fins can be placed downstream of the NO injection point to ensure prompt mixing of the two gas streams. 1, 2, 3, 4, 5, 6, 7, 8, 9, 10, 11, 12, 13, 14, 15 or more blades, plates and/or fins can be used. FIGS. 2A-C provide various views of an exemplary configuration of a mixing device having four blades. FIGS. 3A-C provide various views of an exemplary configuration of a mixing device having three angled fins. FIGS. 4A-C provide various views of an exemplary configuration of a mixing device having eight plates. FIGS. 5A-C provide various views of an exemplary configuration of a mixing device having four curved blades. FIGS. 6A-C provide various views of an exemplary configuration of a mixing device having four curved blades and an injection channel at a tapered section.

When a plurality of blades, plates and/or fins are used in a mixing device, the blades, plates and/or fins can be placed in parallel at the same distance downstream from the NO injection point and/or may be placed in series at various distances downstream from the NO injection point. For example, each blade, plate or fin may be placed 1, 2, 3, 4, 5, 6, 7, 8, 9, 10, 15, 20, 25, 30, 35, 40, 50, 60, 70, 80, 90 or 100 cm downstream from the NO injection point.

The presence of a mixing device can also be used to shorten the distance between the NO injection point and one or more sampling points for monitoring the composition of the combined gas, such as the O₂, NO and NO₂ concentrations. For example, the first sampling point can be located 1, 2, 3, 4, 5, 6, 7, 8, 9, 10, 15, 20, 25, 30, 35, 40, 50, 60, 70, 80, 90 or 100 cm downstream from mixing device. Furthermore, a plurality of sampling points may be used, such as sampling points located at various distances from the NO injection point. 1, 2, 3, 4, 5, 6, 7, 8, 9, 10, 15, 20, 25, 30 or more sampling points may be used. The distance between sampling points can be 1, 2, 3, 4, 5, 6, 7, 8, 9, 10, 15, 20, 25 or 30 cm. The plurality of sampling points can be used to separately analyze the combined gas stream as a function of length down the breathing circuit, or two or more sampling can be combined to provide an average for the composition of the gas.

Furthermore, the location of the point of injection of NO into the fresh gas flow can influence the reduction in NO₂ generation. In exemplary embodiments, the NO injection point can be located where the residence time of the initial high concentration is minimized and/or initial high concentration NO is rapidly dispersed. For example the point of injection of NO *(e.g.,* high concentration NO, 5000 ppm NO) may be located at the center of the annular body or as part of the tapered section, to reduce the amount of time that the NO remains at the initial high concentration. Accordingly, the point of NO injection can be located where the NO will be intermixed quickly with the fresh gas flow thereby minimizing the residence time of the high concentration NO and in turn reducing the NO₂ generated. While not wishing to be bound by any particular theory, it is believed that injecting the NO at a point in which the fresh gas flow has a high velocity will generate less NO₂ than other traditional techniques of injecting NO at the edge (*i.e.* wall) of the tube where fresh gas flow will have a low velocity.

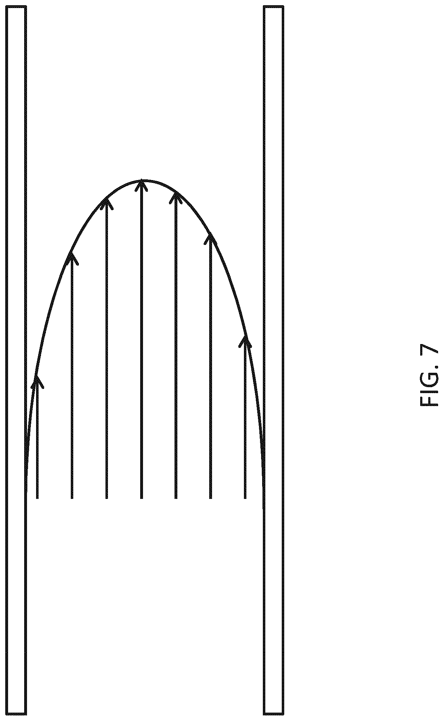

FIG. 7 illustrates an exemplary velocity distribution of a gas flow through a tube. As can be seen from FIG. 7, the gas flow has the lowest velocity closest to the edge boundary (*e.g*. wall of the tube) and has the highest velocity farthest from the edge boundary. Accordingly, in some embodiments the NO is injected at a distance from the edge boundary where the gas velocity is higher than the gas velocity at or close to the edge boundary.

In exemplary embodiments, to reduce NO₂ generation, the point of injection of NO into fresh gas flow can be located where the fresh gas flow is accelerated to the desired velocity. The accelerator may act to increase the fresh gas flow velocity from an inlet end to the outlet end, and the injection port located a distance from the inlet at which the fresh gas flow has increased to an intended velocity. The increase in velocity may be created by conversion of the gas's potential energy to kinetic energy. By way of example, the velocity may be increased by the reducing cross section of the tapered section, as the gas flows from a region of higher pressure to a region of lower pressure. The gas velocity being proportional to the change in cross-sectional area and change in gas density. Of course other techniques for increasing the velocity are envisioned.

Further complicating any potential solutions for minimizing NO₂ generation when injecting NO into the ventilator breathing circuit, ventilators require that any element (e.g., injector module, NO₂ minimization device, etc.) used with the ventilator breathing circuit not cause a substantial change to the ventilator inspiratory flow profile (by way of increased resistance to flow or increased compressible volume). Generally speaking, the allowable pressure drop across the entire breathing circuit can be 6 cm H₂O at 30 SLPM for adults, 6 cm H₂O at 15 SLPM for pediatrics and 6 cm H₂O at 2.5 SLPM for neonates inclusive of ventilator outlet resistance. In light of this, the allowable pressure drop across the diffuser should be minimized. For example, current INOmax DS Injector Module is rated at 1.5 cm H₂O at 60 SLPM. Accordingly, systems and methods of the present technology minimize NO₂ without affecting ventilator performance and/or causing substantial pressure drops, flow profile changes, and introducing substantial compressible volumes, for example, that may affect patient ventilation gas exchange.

Accordingly, in exemplary embodiments, the diffusing device can be configured and dimensioned so that at least the accelerator increase the fresh gas flow impinging velocity at the lowest expected fresh gas flow rate while not causing a substantial pressure drop in the highest peak fresh gas flow, not cause substantial changes to the inspiratory fresh gas flow's flow profile, and not create a substantial compressible volume in the breathing circuit. For example, the mouth and throat diameter may be selected to increase FGF velocity while minimizing delay in pressure changes and gas flow to a patient. To minimize changes to pressure, flow, and compressible volume the diffusing device can include a region for fresh gas flow to bypass the accelerator. For example, the diffuser can include a bypass gap which may be located about the periphery of the diffuser and/or accelerator.

After using the techniques disclosed herein to minimize NO₂ generation in the first phase (*e.g*., rapidly diffusing the NO and fresh gas flow at the point of injection, etc.), the NO may continue to traverse the remaining region of the breathing circuit at, or very close to, the desired set dose *(e.g.,* 1 to 80 ppm NO). As this NO dose, or very close to the desired set dose, traverses the remaining region of the breathing circuit NO₂ may be generated (second phase); however, as described above, using the techniques disclosed herein, the majority of NO₂ that would have been produced will be substantially minimized thereby substantially reducing the total amount of NO₂ generated (e.g., immediate NO₂ generated and latent NO₂ generation).

To further mitigate NO₂ generation, NO may be introduced (e.g., in the ventilator breathing circuit) as close to the patient as technically feasible to reduce the contact time by reducing the time the NO and O₂ are in transit together, thus partly reducing NO₂ formation. NO₂ conversion time is the elapsed time NO and oxygen resides in combination prior to reaching the patient. NO₂ conversion time is therefore a function of ventilator flow rates (inspiratory and expiratory), ventilator I:E ratio, and breathing circuit volume from the point of NO injection to the patient airway end.

However, as explained above, in exemplary embodiments the downstream NO₂ generation (*i.e.* Phase 2) is much less than the NO₂ generation upon injection (Phase 1). Accordingly, in some embodiments the NO-containing gas is injected at a position that is significantly upstream from the patient, such as several feet from the patient, yet the NO₂ can be at an acceptable level (*e.g.* less than 1 ppm). Exemplary NO injection points include those at least 1, 2, 3, 4, 5, 6, 7, 8, 9 or 10 feet upstream from the patient. Such locations can be upstream of a patient "Y" piece, upstream of a humidifier, upstream of a nebulizer or other locations upstream from the patient in the ventilator breathing circuit.

In various embodiments, the second gas stream may be injected at an angle in the range of about 60° to about 120°, or at an angle in the range of about 75° to about 105°, or about 80° to about 100°, or about 85° to about 95°, or at about 90° to the axis of the first gas stream.

An aspect of the present technology relates to an injection device for injecting a high concentration gas into a transverse gas stream.

In one or more embodiments, the device comprises an injection port that injects the second gas stream perpendicularly into the first gas stream.

In various embodiments, a high concentration NO-containing gas may be in the range of greater than 800 ppm NO to about 5000 ppm NO, or about 2000 ppm NO to about 4880 ppm NO, or at about 4880 ppm NO. Exemplary lower limits include about 800 ppm, about 1,000 ppm, about 1,200 ppm, about 1,400 ppm, about 1,600 ppm, about 1,800 ppm, about 2,000 ppm, about 2,200 ppm, about 2,400 ppm, about 2,600 ppm, about 2,800 ppm, about 3,000 ppm, about 3,200 ppm, about 3,400 ppm, about 3,600 ppm, about 3,800 ppm, about 4,000 ppm, about 4,200 ppm, about 4,400 ppm, about 4,600 ppm, and about 4,800 ppm. Exemplary upper limits include about 10,000 pm, about 9,000 ppm, about 8,000 ppm, about 7,000 ppm, about 6,500 ppm, about 6,000 ppm, about 5,500 ppm, about 5,200 ppm, about 5,000 ppm and about 4,900 ppm. The high concentration NO-containing gas may be contained in a pressurized cylinder at a pressure in the range of about 200 psig and about 3000 psig, or in the range of about 2000 psig and about 2400 psig, or about 2200 psig and about 2400 psig. Of course other sources of high concentration NO are envisioned.

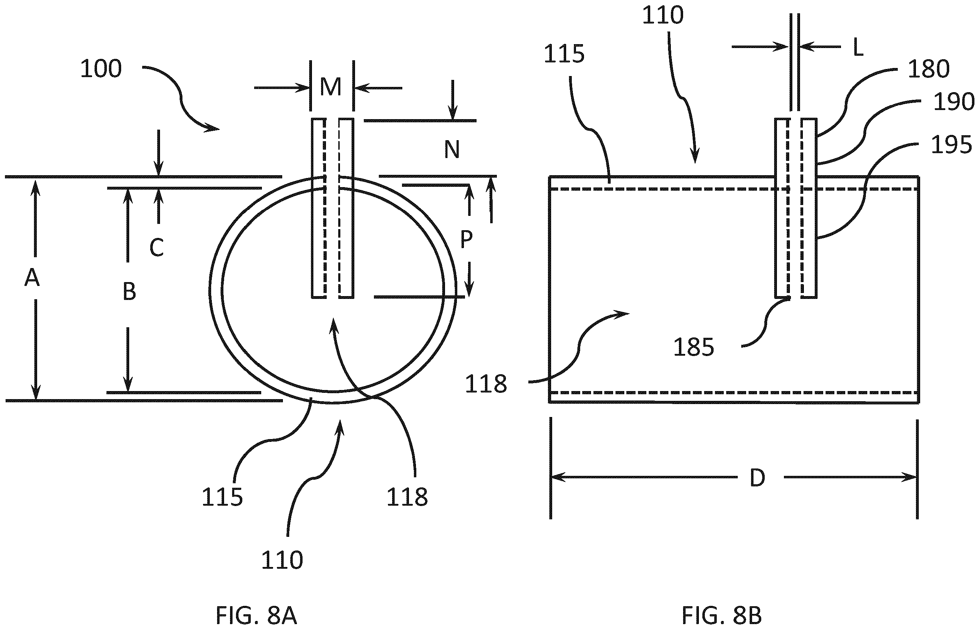

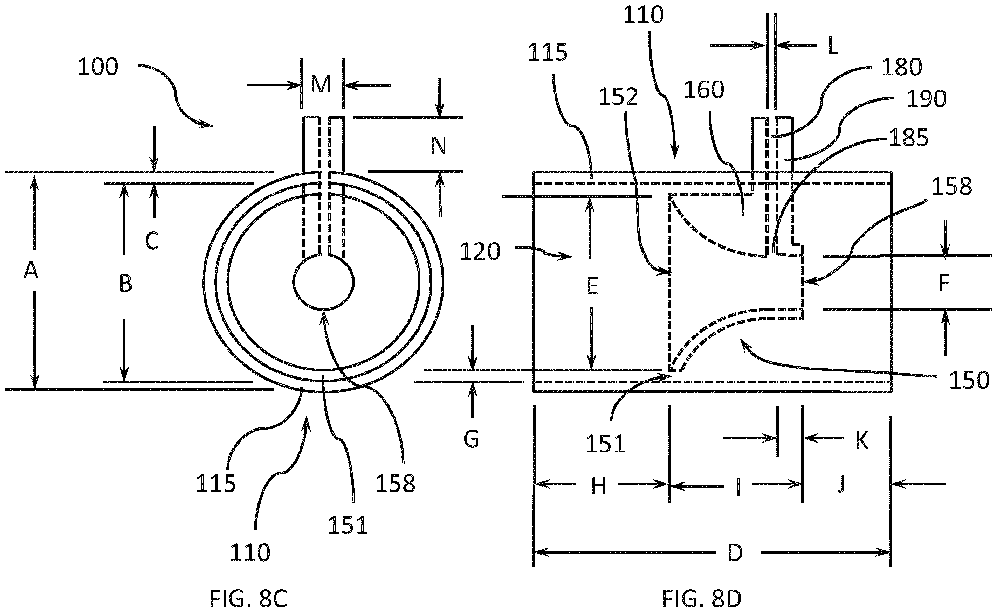

FIGS. 8A-B illustrate an exemplary device for diffusing a high concentration low volume gas flow and a high volume gas flow using the techniques disclosed above.

In one or more embodiments, the diffusing device 100 comprises a body 110 that may be an annular body formed by a cylinder having a wall 115 and a hollow (also referred to as open), internal region 118. The body 110 may be configured and dimensioned to connect to tubing in a ventilator breathing circuit *(e.g.,* 10, 15 and 22 mm), fit into ventilator tubing, or have ventilator tubing fitted into the body. In various embodiments, the inlet end of the device comprises a male connection configured and dimensioned to join to a ventilator tube, and the outlet end comprises a female connection configured and dimensioned to join to a ventilator tube or humidifier chamber inlet. In a non-limiting example, the inlet end of the device comprises a 22 mm (O.D.) male connection, and the outlet end comprises a 22 mm (I.D.) female connection. In addition, in various embodiments the diffusing device 100 can be a component or part of an injector module which couples to a ventilator breathing circuit or component such as humidifier chamber, as is known in the art.

In one or more embodiments, the diffusing device 100 comprises a body 110 that may be rectangular, cubic or other geometric shapes that are configured and dimensioned to connect to tubing in a ventilator breathing circuit *(e.g.,* 10, 15 and 22 mm), and having a hollow internal region. For convenience, in embodiments where the body comprises a cylindrical wall, the body is referred to as an annular body in the specification.

In one or more embodiments, an annular body 110 may have an outside diameter 'A' at an inlet end and/or an outlet end. The outside diameter 'A' may be in the range of about 10 mm (0.394 in.) to about 25mm (1.0 in.), or about 22 mm (0.866 in.), where the ventilator tubing may be fitted around the outside of the inlet end OD and inside the outlet end ID. In various embodiments, a ventilator tube may be connected to an inlet end and/or outlet end of a diffusing device utilizing a friction-fit connection, as would be known in the art. In various embodiments, the OD at the inlet end may be the same or different from the OD of the outlet end.

In one or more embodiments, the annular body may have an inside diameter 'B' at an outlet end and/or an inlet end. The inside diameter 'B' may be in the range of about 10 mm (0.394 in.) to about 25mm (1.0 in.), or about 22 mm (0.866 in.), where the ventilator tubing may be fitted into the inside of the inlet end ID. In various embodiments, a ventilator tube may be connected to an inlet end and/or outlet end of a diffusing device utilizing a friction-fit connection, as would be known in the art. In various embodiments, the ID at the inlet end may be the same or different from the ID of the outlet end.

In one or more embodiments, gas(es) may enter the inlet end of the diffusing device 100 and exit the outlet end of the diffusing device, where the gas(es) may comprise a mixture of breathable gases. In various embodiments, the breathable gases may comprise air, or air and additional oxygen.

In various embodiments, the wall thickness 'C' of a cylindrical wall 115 may be in the range of about 1 mm (0.040 in.) to about 3.175 mm (0.125 in.), or in the range of about 1 mm (0.040 in.) to about 2 mm (0.079 in.), or in the range of about 1.588 mm (0.0625 in.) to about 2.388 mm (0.094 in.).

In one or more embodiments, the diffusing device may have a length 'D' in the range of about 6.35 mm (0.25 in.) to about 41.3 mm (1.625 in.), or in the range of about 22.225 mm (0.875 in.) to about 41.275 mm (1.625 in.), or in the range of about 25.4 mm (1.00 in.) to about 38.1 mm (1.50 in.).

In one or more embodiments, a nipple 190 for attaching a delivery tube to the diffusing device may protrude from the outer surface of the cylindrical wall 115. In various embodiments, the nipple may have a diameter 'M' 4.5 mm diameter (0.177") and protrude from the outer surface of the cylindrical wall 115 a height 'N' 8.7 mm (0.34 in.). In various embodiments, the nipple may comprise hose barbs for affixing a delivery tube.

In one or more embodiments, the device further comprises a projection 195 extending from the inner surface of the cylindrical wall 115. In various embodiments, the projection 195 may extend a radial distance 'P' into the hollow internal region 118. In various embodiments, the projection 195 may extend up to or close to the center of the hollow internal region 118, which would be half of the ID of the wall 115. In various embodiments, distance 'P' is slightly under half the ID so that the NO-containing gas will project out forward from the nozzle orifice to the middle, where the FGF gas velocity is higher than at the inner surface of the cylindrical wall. Accordingly, in various embodiments, the difference between 'P' and 'B'/2 is in the range of from about 0.1 mm to about 5 mm, or about 0.5 mm to about 3 mm. In exemplary embodiments, the difference between 'P' and 'B'/2 is about 1.5 mm, *i.e.* the projection 195 ends about 1.5 mm from the center of the hollow internal region 118. Exemplary differences between 'P' and 'B'/2 can have a lower limit of about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 and about 1.4 mm, and exemplary upper limits can be about 5 mm, about 4.5 mm, about 4 mm, about 3.5 mm, about 3 mm, about 2.5 mm, about 2.4 mm, about 2.3 mm, about 2.2 mm, about 2.1 mm, about 2 mm, about 1.9 mm, about 1.8 mm, about 1.7 mm and about 1.6 mm.

In some embodiments, 'P' is provided as a certain percentage of 'B', such as about 50%, about 49%, about 48%, about 47%, about 46%, about 45%, about 40%, about 40%, about 35%, about 30%, about 25%, about 20%, about 15%, about 10% or about 5% of 'B'. In exemplary embodiments, 'P' is between about 40% and about 45% of 'B'.

In one or more embodiments, the dimensions 'B', 'P', 'L', etc. may be selected to achieve desired relationships between the dimensions and/or desired relationships between gas properties under certain conditions. For example, 'B' and 'L' may be selected such that for a given source gas concentration and a given desired NO dose *(e.g.* 20 ppm), the gas velocity at the lowest expected FGF will be approximately equal to the gas velocity of the NO-containing gas. As another example, 'B' and 'L' may be selected such that for a given source gas concentration, the gas velocity of the FGF will be similar to the gas velocity of the NO-containing gas over a range of desired NO doses *(e.g.* 5 ppm to 80 ppm). As another example, 'B' and 'P' may be selected such that the NO-containing gas projects out forward from the nozzle orifice to a distance from the inner surface of the cylindrical wall, such as at or near center of the hollow internal region 118. As a further example, 'B' and 'P' may be selected such that the NO-containing gas projects out forward from the nozzle orifice to a portion of the FGF having a certain percentage of the peak velocity of the FGF, such as 99%, 98%, 95%, 90%, 85%, 80%, 75%, 70%, 65%, 60%, 55%, 50%, 45%, 40%, 35%, 30%, 25%, 20% or 10% of the peak velocity of the FGF.

In various embodiments, an injection channel 180 leading to an injection port 185 may be formed in the nipple, where the injection channel 180 has an inside diameter of 'L'. In various embodiments, the inside diameter 'L' may be in the range of about 0.8 mm (0.03125 in.) to about 2.4 mm (0.094 in.), or about 1.6 mm (0.0625 in.). Exemplary lower limits include about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm and about 1.6 mm. Exemplary upper limits include about 4.75 mm, about 4.5 mm, about 4 mm, about 3.5 mm, about 3 mm, about 2.5 mm, about 2.4 mm, about 2.29 mm, about 2.2 mm, about 2.1 mm, about 2 mm, about 1.9 mm, about 1.8 mm, about 1.7 mm and about 1.6 mm. The injection channel provides a flow path for delivery of a gas *(e.g.,* NO) to the hollow internal region 118 of the diffusing device 100.

In one or more embodiments, the diffusing device 100 does not comprise a nipple 190 projecting from the body 110, but has a female connector into which a delivery tube may be plugged, where the female connector allows the delivery tube to be connected to and in fluid communication with the injection channel 180. In various embodiments, the ID of the delivery tube and the ID of the injection channel are the same and/or have a uniform cross-sectional area.

In one or more embodiments, the injection port 185 may be an orifice allowing a gas flowing through the injection channel 180 to enter the hollow internal region 118 at an intended rate and/or velocity. In various embodiments, the injection port may be an opening of fixed dimension that may be the same or different from the diameter of the injection channel, which provides an intended flow velocity related to the flow rate. In various embodiments, more than one injection port 185 can be used, such as by having multiple injection ports 185 along the length of the projection 195 and/or by having multiple projections 195, with each projection 195 having one or multiple injection ports 185. As set forth above, the injection ports may inject the NO-containing gas into a portion of the FGF that is a distance from the edge boundary so that the NO-containing gas is injected into FGF having a high velocity, not a portion of the FGF having zero or low velocity. In some embodiments, when more than one injection ports 185 are used, the diameters of the injection ports 185 have smaller diameters than would be used for a single injection port 185 to ensure that the velocity of the NO-containing gas is not reduced and is maintained in proportion to FGF velocity.

In some embodiments utilizing multiple injection ports 185, only one or some of the injection ports 185 may be used at a time, with selection dependent on the set dose of NO. For example, the multiple injection ports 185 can have varying orifice diameters, with a smaller orifice diameter being used for lower set doses of NO and a larger orifice diameter being used for higher set doses of NO. In this way, the ratio of the velocities of the NO-containing gas and the FGF can be maintained at a constant ratio, even with different set doses of NO. In some embodiments, more ports 185 are used at higher set doses of NO and less injection ports 185 are used at lowers set doses of NO, to tailor the velocities of the NO-containing gas and the FGF to the desired ratio. In other embodiments, all of the multiple injection ports 185 may be used concurrently. In various embodiments, multiple injection ports 185 may be multiple proportional control valves as part of the injector module.

In one or more embodiments, a valve (not shown) and/or variable orifice can be in fluid communication with the injection port 185 and/or can be located at the injection port 185. The proportional valve and/or variable orifice can be adjusted to control the velocity of gas being injected from the injection channel 180 into the hollow internal region 118. In various embodiments, the size of a valve orifice and/or variable orifice and velocity of gas being injected through the injection port 185 may be adjusted in relation to the FGF velocity, where the valve and gas velocity may be controlled through a feedback loop. In various embodiments, the feedback loop may comprise a flow sensor capable of measuring fresh gas flow in the breathing circuit, where the flow sensor may be in electrical communication with a control module that controls the dosage of NO fed into the diffusing module 100 through the injection channel 180 and the valve and/or variable orifice by adjusting the valve and/or variable orifice. In one or more embodiments, the diffuser and flow sensor capable of measuring fresh gas flow are incorporated into a single piece, such as being integral to an injector module.

In one or more embodiments, the diffusing module 100 comprises a proportional control valve, an NO flow sensor and a FGF flow sensor for measuring the fresh gas flow in the breathing circuit and delivering a flow of NO-containing gas that is proportional to the FGF to provide the desired set dose of NO. In such embodiments, the proportional control valve and/or flow sensor can be eliminated from the control module. Such a configuration can eliminate a compressed gas volume between the control module and the diffuser, as the proportional valve within the diffusing module 100 is used as the primary valve for regulating the flow of the NO-containing gas into the breathing circuit. While not wishing to be bound by any particular theory, it is believed that having both a proportional valve in the diffusing module 100 and a proportional valve in the control module can result in compressed gas being stored within the injection channel and NO delivery tube at the end of each inspiratory cycle, and that this compressed gas may then decompress, allowing a quantity of NO-containing gas to enter the breathing circuit and causing over delivery of NO. This potential problem can be magnified with high concentration NO, due to the decreased delivery volume. Accordingly, substituting a proportional control valve in the diffusing module 100 for the proportional control valve in the control module can reduce or eliminate the impact of this potential problem.

In one or more embodiments, the NO-containing gas is injected into the FGF as a plurality of pulses from one or more injection ports 185. The plurality of pulses can be used to provide a higher velocity of the NO-containing gas than if the flow of the NO-containing gas was constant. By providing pulses *(e.g.* NO flow OFF-ON-OFF-ON), a higher instantaneous NO volumetric flow rate can be provided with a corresponding increase in instantaneous NO velocity, without providing a higher average volumetric flow rate than needed to provide the desired NO concentration in the combined gas stream. As an example, if the system was to detect low FGF bias flow *(e.g.* 0.5 SLPM), the NO can be delivered as a plurality of high-velocity pulses to maintain the correct quantity of NO-containing gas volume during this phase. In this way, the NO delivery system can utilize pulse width modulation of NO flow *(e.g.* during expiratory bias flow) to maintain a higher gas velocity of NO in proportion to FGF gas velocity, while maintaining the desired average NO flow rate or set dose concentration.

During expiratory phase only delivery of pulsatile high peak flow to increase the NO exit velocity. In order to maintain the correct quantity of gas volume during this phase. The pulsatile flow would be Off-ON-Off-On to meet the average flow rate required to meet set Dose. Pulse width modulation of NO flow.

Aspects of the technology also relate to method of diffusing a high concentration gas into a transverse gas stream comprising passing at least a portion of a first gas longitudinally through a hollow internal region of a body having an inner surface surrounding the hollow internal region, and passing a second gas stream through an injection channel to an injection port projecting into the hollow internal region of the body, wherein the second gas stream enters and at least partially diffuses with the first gas stream within the hollow internal region.

FIGS. 8C-D illustrate another exemplary device for diffusing a high concentration low volume gas flow and a high volume gas flow using the techniques disclosed above. Of course, other configurations capable of diffusing a high concentration low volume gas flow and a high volume gas flow using the above techniques are envisioned. The dimensions are exemplary for a 22 mm nominal diffuser for use with adult breathing circuits/fittings. It should be noted that the non-limiting examples of dimensions and/or configurations are intended for standard adult breathing circuits, and the dimensions and proportions of the device may be adjusted for applications involving standard neonate breathing circuits, standard pediatric breathing circuits, or other non-standard-sized breathing circuits without undue experimentation.

In one or more embodiments, the diffusing device 100 comprises an annular body 110 that may be a cylinder having a wall and a hollow internal region. The body may be configured and dimensioned to connect to tubing in a ventilator breathing circuit (10, 15 and 22 mm), fit into ventilator tubing, or have ventilator tubing fitted into the body. In various embodiments, the inlet end of the device comprises a male connection configured and dimensioned to join to a ventilator tube, and the outlet end comprises a female connection configured and dimensioned to join to a ventilator tube or humidifier chamber inlet. In a non-limiting example, the inlet end of the device comprises a 22 mm (O.D.) male connection, and the outlet end comprises a 22 mm (I.D.) female connection. In addition, the diffusing device 100 can be a component or part of an injector module which couples to a ventilator breathing circuit, as is known in the art.

In one or more embodiments, the annular body 110 may have an outside diameter 'A' at an inlet end and/or an outlet end. The outside diameter 'A' may be in the range of about 10 mm (0.394 in.) to about 25mm (1.0 in.), or about 22 mm (0.866 in.), where the ventilator tubing may be fitted around the outside of the inlet end OD and inside the outlet end ID. In various embodiments, a ventilator tube may be connected to an inlet end and/or outlet end of a diffusing device utilizing a friction-fit connection, as would be known in the art.

In one or more embodiments, the annular body may have an inside diameter 'B' at an outlet end and/or an inlet end. The inside diameter 'B' may be in the range of about 10 mm (0.394 in.) to about 25mm (1.0 in.), or about 22 mm (0.866 in.), where the ventilator tubing may be fitted into the inside of the inlet end ID. In various embodiments, a ventilator tube may be connected to an inlet end and/or outlet end of a diffusing device utilizing a friction-fit connection, as would be known in the art.

In one or more embodiments, gas(es) may enter the inlet end of the diffusing device 100 and exit the outlet end of the diffusing device, where the gas(es) may comprise a mixture of breathable gases. In various embodiments, the breathable gases may comprise air, or air and additional oxygen.

In various embodiments, the wall thickness 'C' of the diffusing device 100 may be in the range of about 1 mm (0.040 in.) to about 3.175 mm (0.125 in.), or in the range of about 1 mm (0.040 in.) to about 2 mm (0.079 in.), or in the range of about 0.0625 to about 0.094.

In one or more embodiments, the diffusing device may have a length 'D' in the range of about 6.35 mm (0.25 in.) to about 41.3 mm (1.625 in.), or in the range of about 22.225 mm (0.875 in.) to about 41.275 mm (1.625 in.), or in the range of about 25.4 mm (1.00 in.) to about 38.1 mm (1.50 in.).

In one or more embodiments, the device further comprises a tapered section 150 comprising a wall, which may have a truncated cone, a funnel, or a bell shape, where the tapered section 150 narrows from an inside diameter 'E' at a first (inlet) end to an inside diameter 'F' at a second (outlet) end opposite the first end, wherein the opening at the first (inlet) end has a larger diameter than the opening at the second (outlet) end. In various embodiments, the first end having a larger diameter is a mouth 152, and the second end having the smaller diameter is a throat 158.

In one or more embodiments, an accelerator may comprise a tapered section or a bi-directional tapered section.

In various embodiments, the inside diameter 'E' at the mouth 152 may be in the range of about 14 mm (0.511 in.) to about 18 mm (0.709 in.), or about 16.03 mm (0.631 in.).

In various embodiments, the inside diameter 'F' at the throat 158 may be in the range of about 3.17 mm (0.125 in.) to about 9.5 mm (0.375 in.), or about 6.35 mm (0.250 in.).

In one or more embodiments, the tapered section 150 may have a length 'I' from the leading edge of the mouth 152 to the trailing edge of the throat 158. In various embodiments, the length 'I' of the tapered section 150 may be in the range of about 8 mm (0.315 in.) to about 13 mm (0.519 in.), or about 10.3 mm (0.405 in.).

In one or more embodiments, the inside surface of the tapered section forms a sharp corner at the leading edge of the mouth 150, so there are no flat surfaces perpendicular to the axis of the tapered section. In various embodiments, the wall of the tapered section may have a thickness in the range of about 1 mm to about 2 mm or about 1.5 mm.

In one or more embodiments, the tapered section 150 may be located inside the body 110 of the diffusing device 100. In various embodiments, the tapered section may be suspended from a cylindrical wall 115 of the annular body 110 by a support 160, wherein the support 160 may extend from an inner surface of the cylindrical wall 115 into the open internal region 118. In various embodiments, the annular body 110, tapered section 150, and support joining the tapered section 150 to the annular body may be one integral piece, where the annular body 110, tapered section 150, and support 160 are molded as a single piece, so the components comprise a single unitary construction. In various embodiments, the tapered section 150 and the annular body 110 are coaxial. In one or more embodiments, the projection 195 may form the support 160 by interconnecting the body 110 and the tapered section 150.

In one or more embodiments, there may be a gap 151 between the rim of the mouth 152 and the inside surface of the cylindrical wall 115, where the gap 151 has a size 'G' in the range of about 0.5 mm (0.02 in.) to about 3 mm (0.118 in.), which provides an opening around the rim of the mouth 152. In various embodiments, the opening allows at least a portion of the incoming gas(es) to by-pass the tapered section 150 by flowing along the periphery of the internal region and around the tapered section 150.

In one or more embodiments, the opening has a cross-sectional area in the range of about 9.5% to about 19.0% of the cross-sectional area of the internal region

In one or more embodiments, the gap 151 has a cross-sectional area in the range of about 15% to about 35% of the cross-sectional area of the internal region where the internal region defined as B diameter is 20 mm.

In one or more embodiments, the tapered section 150 may be a distance 'H' from the leading edge of the annular body 110. In various embodiments, the distance 'H' from the leading edge of the annular body 110 may be in the range of 3.175 mm (0.125 in.) to about 12.7 mm (0.50 in.). In various embodiments, dimension H may be reduced to thereby minimize the size and weight of the device.

In one or more embodiments, the tapered section 150 may be a distance 'J' from the trailing edge of the annular body 110. In various embodiments, the distance 'J' from the trailing edge of the annular body 110 may be in the range of 3.175 mm (0.125 in.) to about 12.7 mm (0.50 in.), In various embodiments, dimension J may be reduced to thereby minimize the size and weight of the device.

In one or more embodiments, a nipple 190 for attaching a delivery tube to the diffusing device may protrude from the outer surface of the cylindrical wall 115. In various embodiments, the nipple may have a diameter 'M' of about 4.5mm diameter (0.177 in.) and protrude from the outer surface of the cylindrical wall 115 a height 'N' of about 8.7mm (0.34 in.).

In various embodiments, an injection channel 180 leading to an injection port may be formed in the nipple, where the injection channel 180 has an inside diameter of 'L'. In various embodiments, the inside diameter 'L' may be in the range of about 0.8 mm (0.03125 in.) to about 2.4 mm (0.094 in.), or about 1.6 mm (0.0625 in.).

In one or more embodiments, the opening forming the injection port 185 at the internal end of the injection channel 180 may be located proximal to region where fresh gas velocity is maximized in diffuser device *(e.g.* a distance 'K' from the outlet end of the tapered section 150. In various embodiments, the distance 'K' may be in the range of about 2 mm (0.787 in.) to about 5 mm (0.197 in.), or about 3 mm (0.118 in.) from the outlet end of the tapered section 150).

In one or more embodiments, the NO injection port may terminate at the throat wall, or an extension tube may project further into the throat from the internal surface of the tapered section. In various embodiments, the extension tube may project into center of the throat.

In one or more embodiments, the injection port may be 6.81 mm from the leading edge of the tapered section.

In one or more embodiments, the tapered section may be suspended within a hollow cylindrical portion of a housing, wherein the housing is adapted to connect to ventilator tubing. In various embodiments, the housing may have a shape other than cylindrical or annular while having an inlet and outlet configured and dimension to connect to suitable ventilator tubing. For example a rectangular housing of a diffusing device may have cylindrical inlet and outlet openings with an I.D. to connect to tubing.

In one or more embodiments, a diffusing device may be utilized in a ventilator circuit, with a nasal cannula, or with a face mask.

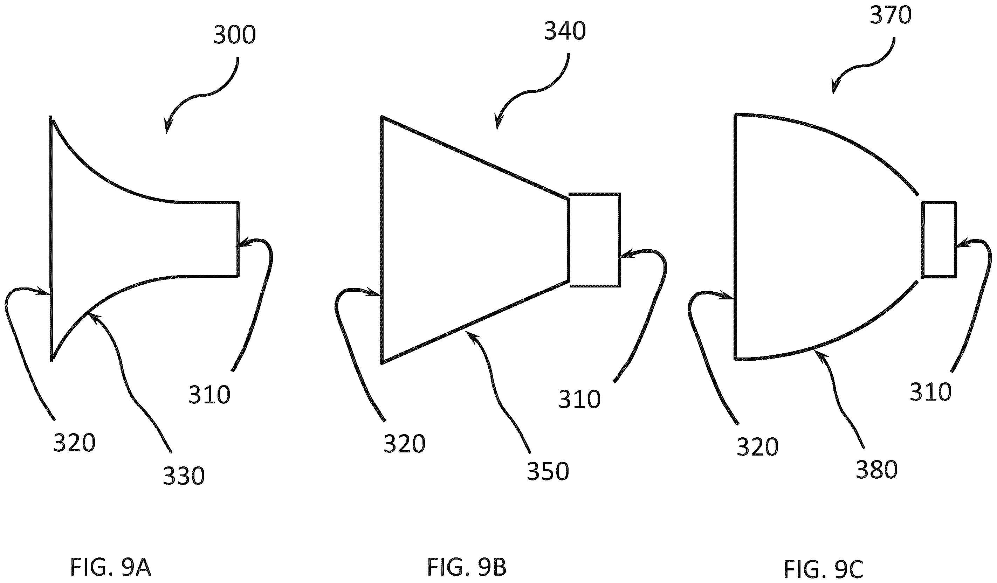

FIG. 9A illustrates an exemplary embodiment of a tapered section 300 having a funnel shape.

In one or more embodiments, the funnel shaped tapered section 300 has an internal surface that is convex, and directs gas(es) entering the mouth 320 towards the throat 310. In various embodiments, the convex contour of the internal surface may have a constant curvature or a changing curvature.

FIG. 9B illustrates an exemplary embodiment of a tapered section 340 having a cone shape.

In one or more embodiments, the cone shaped tapered section 340 has an internal surface that is straight from the mouth 320 of the tapered section 340 to the throat 310, and directs gas(es) entering the mouth 320 towards the throat 310.

FIG. 9C illustrates an exemplary embodiment of a tapered section 370 having a bell shape, where the bell shape may have constant curvature or a changing curvature.

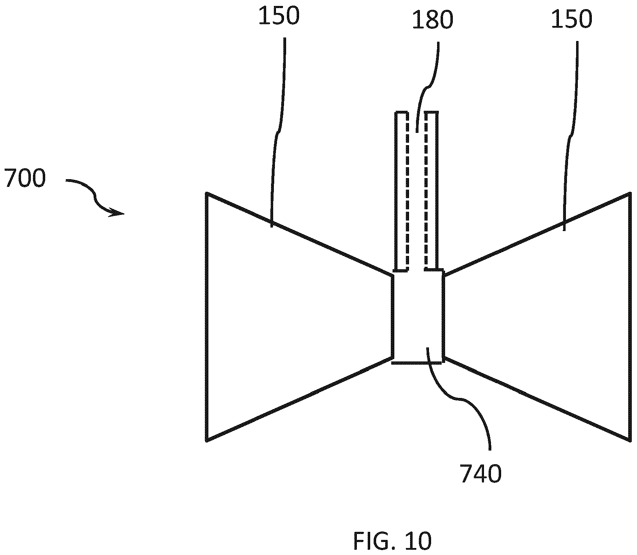

In various embodiments, a tapered section, as depicted in 300, 340, and 370 may be adjoined throat-to-throat to provide a bi-directional tapered section to allow for insertion and use in a ventilation circuit in either orientation. FIG. 10 illustrates an exemplary embodiment of a bi-directional tapered section. A bi-directional tapered section 700 may comprise two tapered sections 150 coupled at their throats, where the injection valve provides for injection of a gas at the narrowest portion of the bidirectional tapered section 700. In various embodiments, the two tapered sections may be coupled at a throat comprising a cylindrical section 740. In various embodiments, the injection port would be located where the two tapered sections join, and the FGF velocity should be at a maximum at the lowest expected FGF rate. In some embodiments, a tapered section is utilized in environments in which the FGF rate is expected to be low, *e.g.* less than 2 SLPM.

In one or more embodiments, the bell shaped tapered section 370 has an internal surface that is concave, and directs gas(es) entering the mouth 320 towards the throat 310.

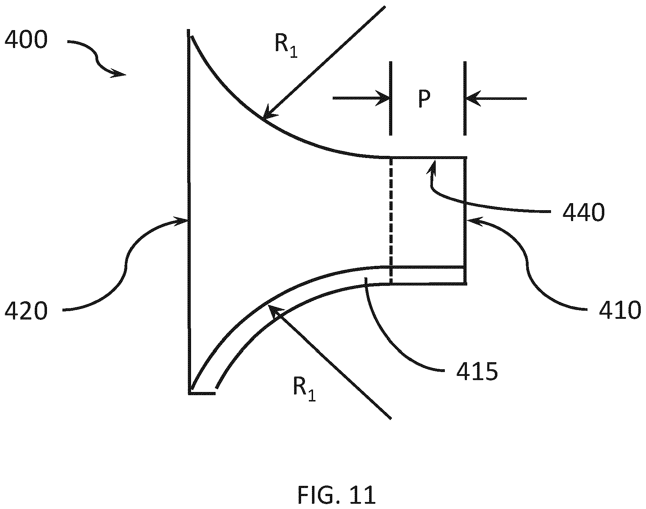

FIG. 11 illustrates an exemplary tapered section 400 depicting a contour of an inside surface of a tapered section wall 415.

Principles and embodiments of the present invention also relate to diffusing device comprising a tapered section 400 comprising a decreasing cross-sectional area that increases the velocity of the gas flow past the injection port and exiting the throat, so a high concentration gas is quickly dispersed and diffused with the ventilator gas.

In one or more embodiments, the tapered section 400 can be an axially-symmetrical tube with a variable cross-sectional area, where area is decreasing from the mouth area to the throat area. In various embodiments, the wall 415 may have a straight, parabolic, hyperbolic, catenoidal, or funnel contour.

In one or more embodiments, the tapered section 400 may comprise a cylindrical section 440 with a constant diameter and cross-sectional area that extends a length 'P' from the point that the cross-sectional area is at a minimum, and/or the slope of the tapered section becomes 0 (zero) (*i.e.,* horizontal).

In various embodiments, the tapered section creates an increasing pressure gradient, so flow or boundary separation cannot occur because of the favorable pressure gradient. The avoidance of boundary separation also avoids reverse-flow regions and vortices that may deplete the energy of the gas flow and increase flow resistance. The pressure drop for a volumetric flow rate of 60 SLPM may be approximately 0.65 cm H₂O, and at 30 SLPM may be approximately 0.16 cm H₂O.

In one or more embodiments, the contour of the tapered section wall 430 has a constant curvature with a radius R₁, where R₁ may be in the range of 7.5 mm (0.296 in.) to about 8.3 mm (0.328 in.), or about 7.6 mm (0.299 in.).

An aspect of the present technology relates to a method of diffusing a high concentration gas into a transverse gas stream.

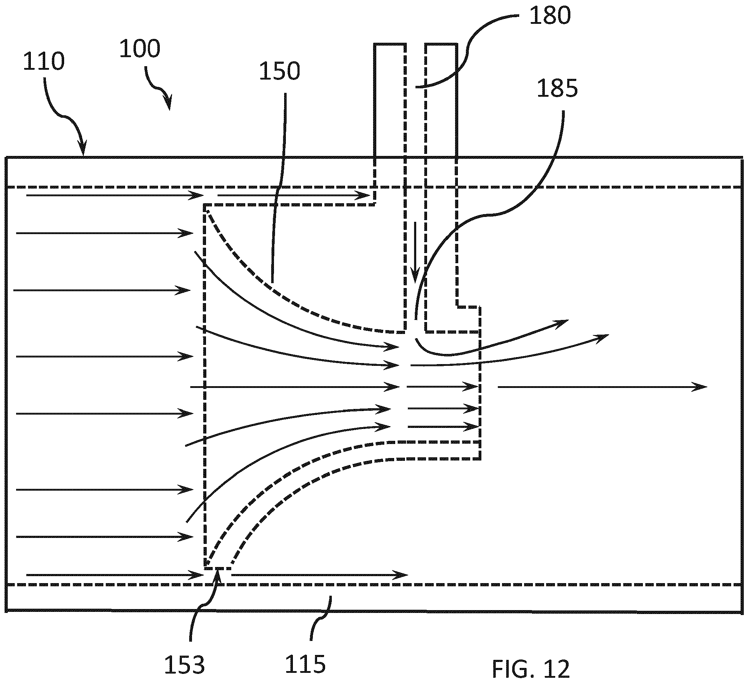

FIG. 12 illustrates an exemplary embodiment of a second gas passing through an injection channel 180 into a first gas passing through a tapered section 150. (Gas flows are indicated by straight and curved arrows.)

In one or more embodiments, at least a portion of a first gas enters a diffusing device 100 and passes through a tapered section 150 comprising a wall 155 having a thickness, an outer surface and an inner surface, an inlet end having a first diameter, and an outlet end having a second diameter opposite the inlet end, wherein the second diameter is smaller than the first diameter; and passing a second gas stream through an injection channel 180 to an injection port 185 in the inner surface of the tapered section 150. In various embodiments, the second gas stream enters and at least partially diffuses with the first gas stream within the tapered section 150. In various embodiments, the injection of the second gas at an intended flow rate and velocity into the stream of the first gas creates sufficient diffusing at the point contact or confluence of the two gas streams. In various embodiments, the intended volumetric flow rate of the second gas (NO at 1-80 ppm dose) may be in the range of about 0.1 SMLPM to about 33.3 SMLPM for 4880 ppm NO, where the volumetric flow rate of the second gas (NO) is proportional to the volumetric flow rate of the first gas (FGF) when the first gas flow rate is in the range of about 0.5 SLPM to about 2.0 SLPM.

In one or more embodiments, at least a portion of the first gas passes around at least a portion of the outer surface of the tapered section, wherein the tapered section 150 is within an annular body 110 having an outer surface and an inner surface, and an inside diameter that is larger than the first diameter of the tapered section. In various embodiments, at least a portion of the first gas passes through the gap 151 between the rim 153 of the mouth 152 and the inside surface of the cylindrical wall 115.

In one or more embodiments, the first gas is a breathable gas comprising molecular N₂ and molecular O₂, and the second gas comprises molecular NO and molecular N₂.

In one or more embodiments, the first gas is provided by a ventilator at a flow rate in the range of about 0 liters per minute (SLPM) to about 120 liters per minute (SLPM). In some instances, as described herein, during expiratory flow there may be flows in the range 0.5 SLPM to 2 SLPM that may result in higher NO₂ being generated. Accordingly, in at least some instances, the disclosed techniques may be directed towards these lower flow rates.

In various embodiments, the concentration of NO in the second gas is in the range of greater than 800 ppm to about 5000 ppm, or about 2000 ppm to about 4880 ppm, or about 4800 ppm.

In one or more embodiments, the flow rate of the second gas is linearly proportional to the flow rate of the first gas.

In one or more embodiments, the second gas stream initially enters the first gas stream at an angle in the range of about 60° to about 120°, or at an angle in the range of about 75° to about 105°, or about 80° to about 100°, or about 85° to about 95°, or at about 90° to the axis of the first gas stream. In various embodiments, the second gas may be injected perpendicularly to the first gas stream, where the two perpendicular gas streams act to impart turbulence at the point of contact, to reduce NO₂ levels to a value equal to or less than the amount generated by the current 800ppm therapy.

Without being limited by theory, it is believed that sufficient diffusion results when FGF is impinged by intersecting NO flow, where the NO and FGF have sufficient velocity at ventilator bias flows. In addition, a short annular outlet just after the point of NO injection may allow for a quick divergence of the once compressed FGF gas within the tapered section, now combined with NO, to exit abruptly and freely diffuse with bypass flow around the tapered section.

In one or more embodiments, the second gas exits the injection port 185 at a flow rate in the range of about 0.1 milliliters per minute (SMLPM) to about 6.3 SLPM, or about 0.05 milliliters per minute (SMLPM) to about 2 SLPM, or about 1.0 milliliters per minute (SMLPM) to about 1 SLPM. A gas flow rate of 2 SLPM has a velocity of approximately 0.42 meters/sec. through an injection channel and injection port with a 0.16 cm I.D. A gas with this velocity would not experience noticeable compression at this velocity when passing through the diffusing device, which is less than 0.2 x the speed of sound *(i.e.,* Mach Number < 0.2). A gas flow rate of 0.5 SLPM has a velocity of approximately 0.10 meters/sec. through an injection channel and injection port with a 0.16 cm I.D. It can be helpful to manage NO₂ conversion during periods of very low ventilator flow rates (e.g., bias flow during exhalation ≤2 SLPM), increased oxygen concentrations (FiO₂ ≥ 60%), and higher NO set dosage (≥20 ppm).

In one or more embodiments, the velocity of the first gas is greater at the second diameter of the tapered section 150 than the velocity of the first gas at the first diameter of the tapered section 150.

In one or more embodiments, the velocity of the first gas is greater at the second diameter of the tapered section than the velocity of the first gas at the first diameter of the tapered section, wherein the second gas enters the first gas at a point of greater velocity. In various embodiments, the tapered section generates an increase gas velocity and pressure gradient towards the middle of the annular body, such that the highest gas velocity is along the axis of the tapered section 150. For example, a reduction of the tapered section 150 I.D. from 1.6 cm at the mouth to 0.635 cm at the throat would result in an increase in the first gas velocity. In some instances, the ratio of inlet to outlet gas velocities is proportional to the ratio of inlet to outlet areas.

As can be seen in FIG. 12, the second gas enters the first gas at the injection port 185, which is closer to the throat of the tapered section 150, and where the velocity of the first gas flow has increased compared to the first gas velocity at the mouth of the tapered section.

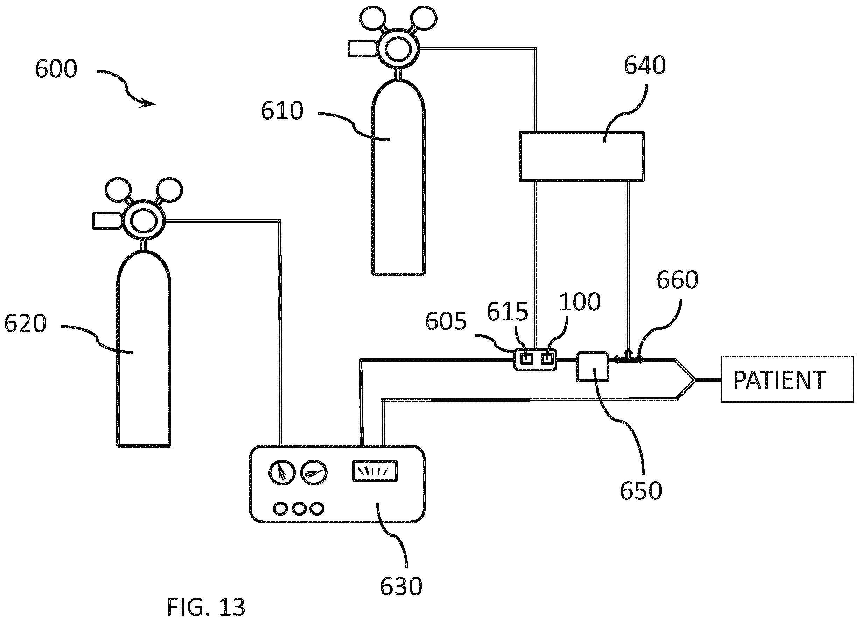

FIG. 13 illustrates an exemplary embodiment of a diffusing device 100 inserted into a ventilator circuit 600. In various embodiments, the ventilator system may provide elevated (>21%) fractional inspired oxygen (FiO₂) concentrations along with NO doses to mechanically ventilated patients. Oxygen concentration in patient ventilator circuits may range from medical air (21% O₂) to medical oxygen (100% O₂), but are generally elevated to 60% for patients receiving INO therapy. The NO in a high concentration NO gas source 610 may be diluted with nitrogen N₂.

In one or more embodiments, a diffusing device 100 *(e.g.,* as a component in an injector module 605, downstream of a flow sensor 615 capable of measuring fresh gas flow in the breathing circuit, etc.) may be connected to and in fluid communication with ventilator tubing coming from a ventilator 630. The ventilator may be connected to and in fluid communication with a fresh gas source 620. The diffusing module 100 may also be connected to and in fluid communication with a control module 640 that controls the dosage of NO fed into the diffusing module 100. The control module 640 may be connected to and in fluid communication with a NO gas source 610. In various embodiments, the fresh gas source 620 and NO gas source 610 may have regulators to control the pressure from the cylinders. In various embodiments, the diffusing device may be connected to and in fluid communication with a humidifier 650 that adds water vapor content to the inspiratory gas flow to the patient. In various embodiments, the distance from the diffusing device 100 to the patient may be approximately 1 meter. In various embodiments, the humidifier may have a compressible volume of about 280 ml. In various embodiments, the diffusing device 100 and the flow sensor 615 are integral to the injector module 605.

In one or more embodiments, the diffusing device diffuses the incoming fresh gas flow from the ventilator 630 and fresh gas source 620 with the incoming NO-containing gas from the NO gas source 610 flowing through the control module 640. The gas flow being delivered to the patient may be sampled at a sampling tee 660 inserted down stream from the humidifier 650 and/or diffusing device 100. In various embodiments, NO, NO₂, and/or O₂ concentrations may be monitored before reaching the patient. The sampling tee 600 can be placed at various positions in the breathing circuit, depending on how quickly the NO-containing gas and FGF combine to provide a homogenous gas stream at the set dose. Furthermore, a plurality of sampling points may be used, such as sampling points located at various distances from the NO injection point. 1, 2, 3, 4, 5, 6, 7, 8, 9, 10, 15, 20, 25, 30 or more sampling points may be used. The distance between sampling points can be 1, 2, 3, 4, 5, 6, 7, 8, 9, 10, 15, 20, 25 or 30 cm. The plurality of sampling points can be used to separately analyze the combined gas stream as a function of length down the breathing circuit, or two or more sampling can be combined to provide an average for the composition of the gas.

As explained in the Examples below, an increase in temperature has surprisingly been found to decrease the amount of NO₂ that is generated under otherwise similar conditions. Accordingly, embodiments of the present technology also relate to minimizing NO₂ generation by heating one or more portions of the NO delivery system and/or ventilator circuit. While not wishing to be bound by any particular theory, it is believed that an increase in gas temperature can increase the available kinetic energy with the gas molecules, which can promote initial mixing resulting in further NO₂ reduction.

For example, a heating element may be added to the NO delivery system, the tubing from the NO delivery system to the injector module, the injector module and/or the tubing of the inspiratory limb of the ventilator circuit, and/or may be placed at any other location upstream, downstream or at the point of injection. The heating element may be a heated humidifier or may be a dedicated heating component. Exemplary heating elements include, but are not limited to, a thermoelectric cooling device or a resistive heating element. A heating element in the NO delivery system can help minimize NO₂ generated internally within the NO delivery system. Likewise, heating elements placed in, and/or in thermal communication with, the tubing that deliver the NO to the injector module and from the injector module to the patient can help minimize NO₂ generation at those points.

In various embodiments, the heating element can heat the NO source gas and/or the combined NO and FGF to a desired temperature. Exemplary temperatures include, but are not limited to, about 25 °C, about 26 °C, about 27 °C, about 28 °C, about 29 °C, about 30 °C, about 31 °C, about 32 °C, about 33 °C, about 34 °C, about 35 °C, about 36 °C, about 37 °C, about 38 °C, about 39 °C, about 40 °C, about 45 °C or about 50 °C.

### EXAMPLES

The present technology is further described by means of the examples, presented below. The use of such examples is illustrative only and in no way limits the scope and meaning of the technology or of any exemplified term.

### EXAMPLE 1 - NO₂ GENERATION SYSTEM COMPARISON

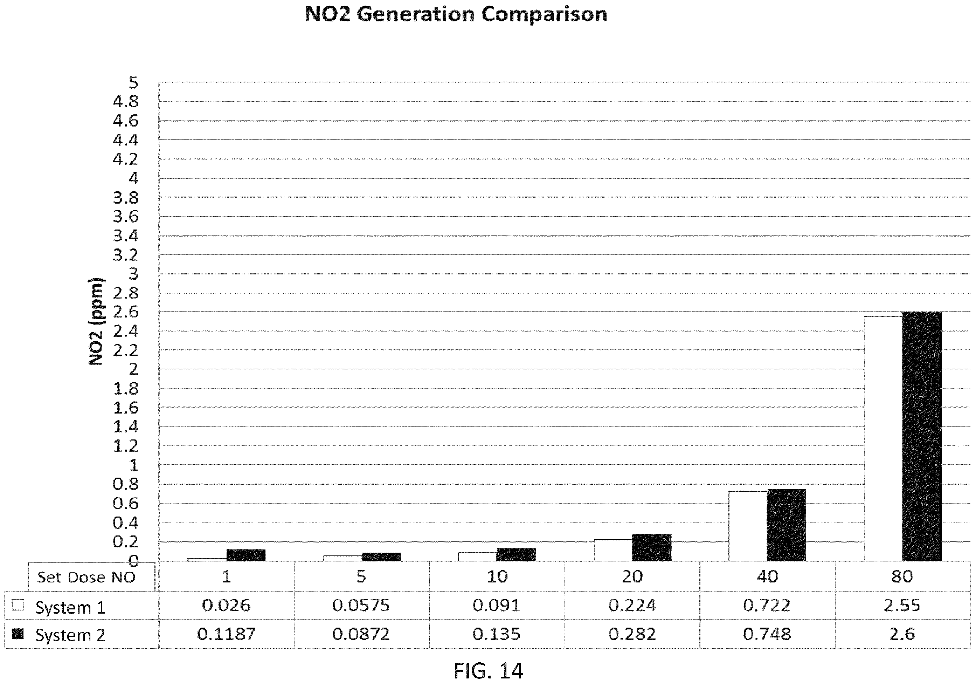

A NO delivery system utilizing a high NO source concentration (e.g. 4880 ppm) and an injector module with an exemplary diffuser as described herein *(e.g.* a diffuser as shown in FIGS. 8A-B) was compared to a conventional NO delivery system utilizing a low NO source concentration (e.g. 800 ppm) and a conventional injector module. The FGF was provided by a neonatal ventilator with exemplary ventilation parameters (e.g. respiratory rate of 40, tidal volume of 30 ml, FiO₂ of 60%, 0.5 SLPM bias flow, etc.). As can be seen from FIG. 14, the high NO source concentration system utilizing a diffuser (System 2) produced a comparable amount of NO₂ as the conventional NO delivery system at a lower NO source concentration (System 1), despite a significantly higher NO source concentration.

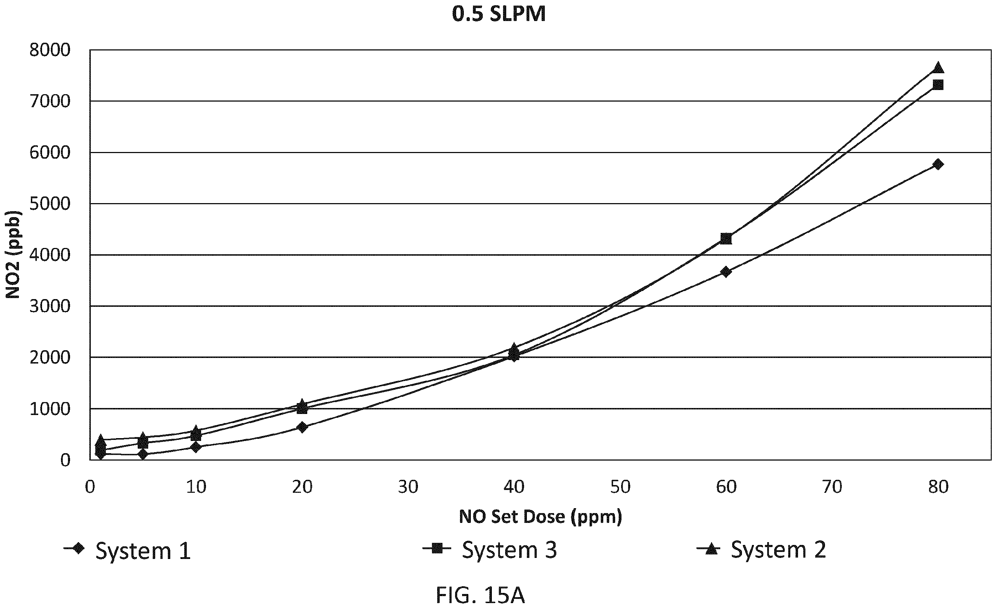

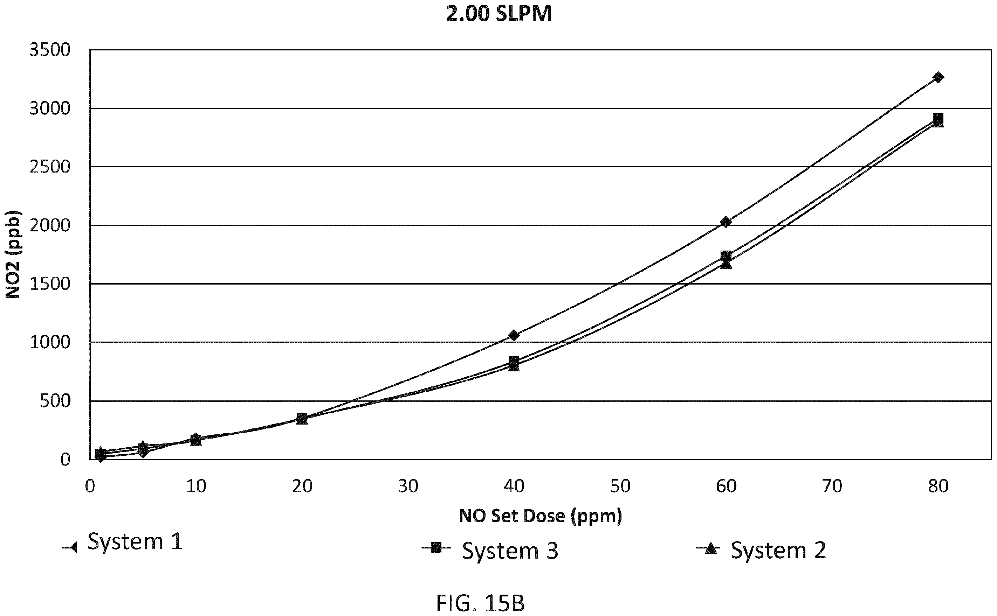

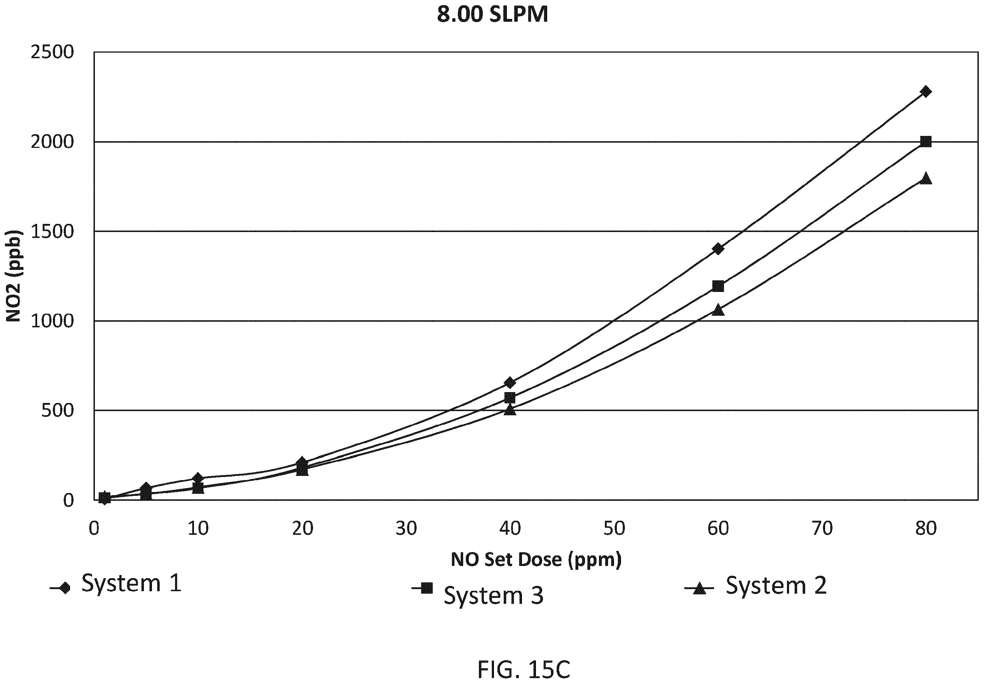

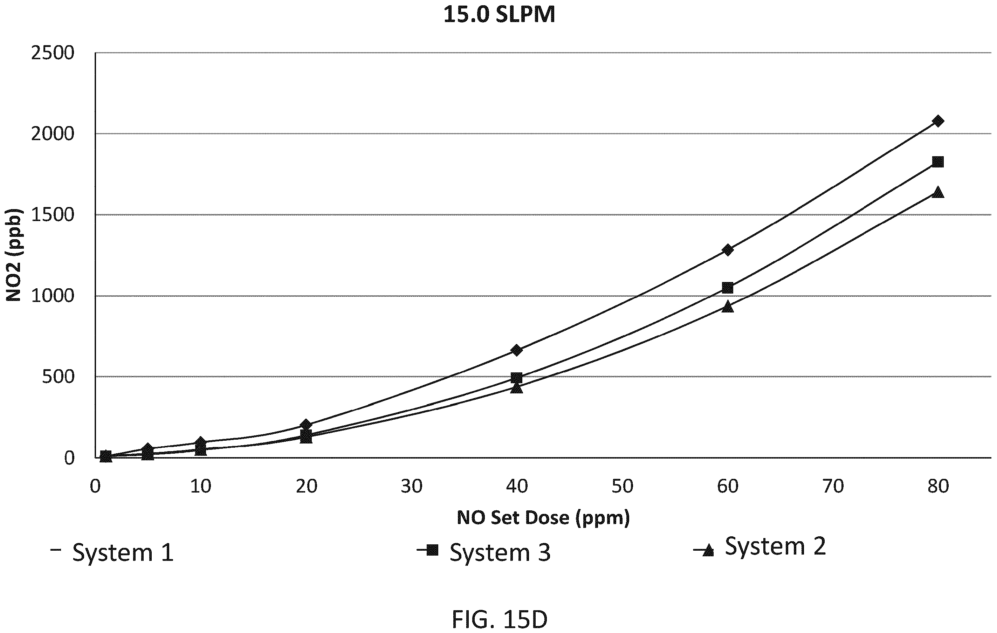

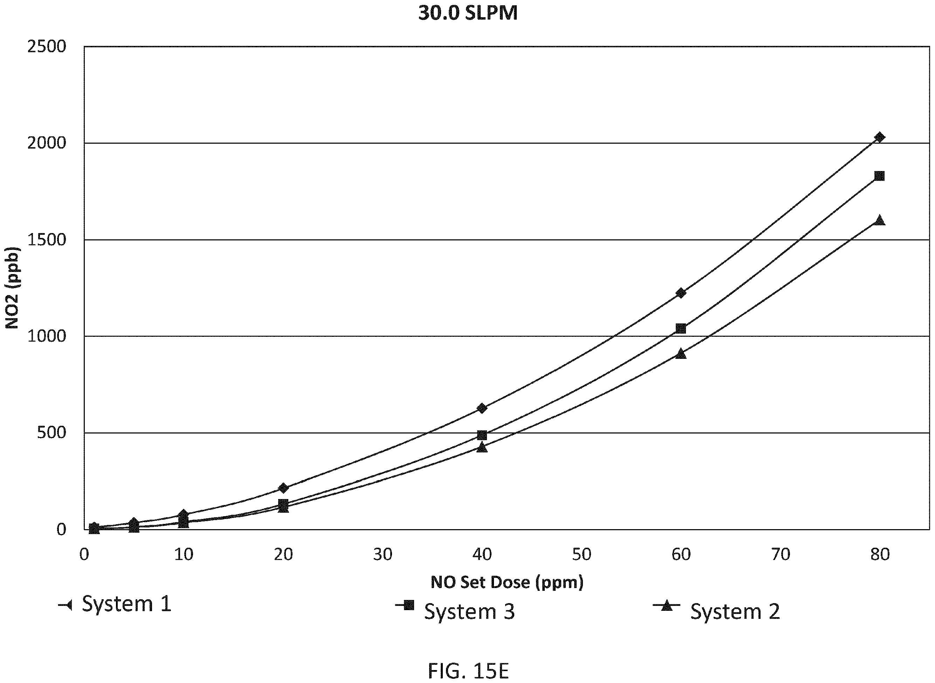

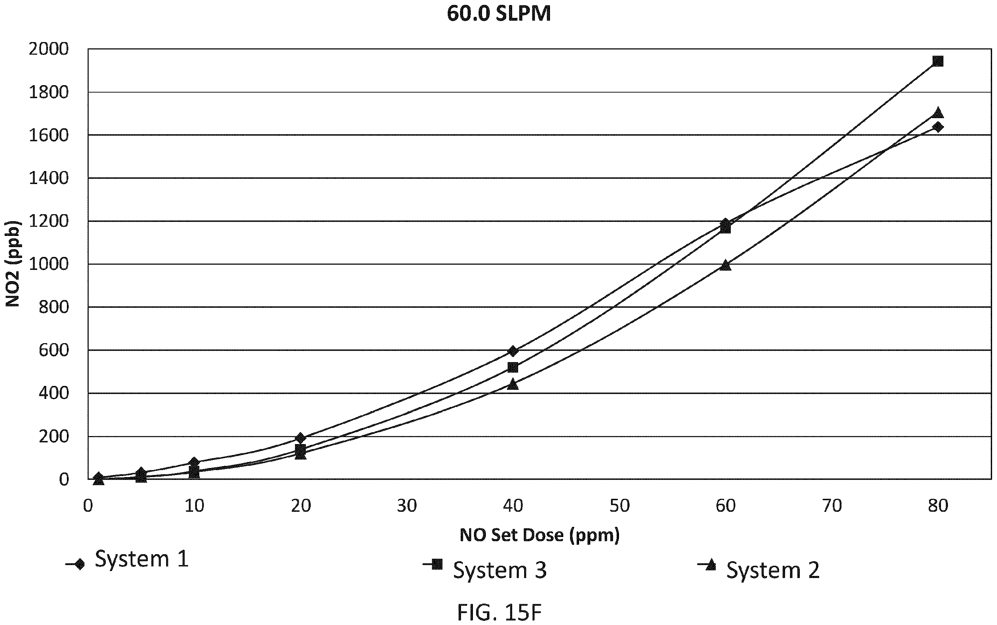

System 1 and System 2 were also compared to a NO delivery system utilizing a high NO source concentration (e.g. 4880 ppm) and an injector module with an exemplary accelerator as described herein *(e.g.* an accelerator as shown in FIGS. 8C-D), which is designated System 3. FIGS. 15A-F show the NO₂ produced for each system at various NO set doses and FGF flow rates. As can be seen from FIGS. 15A-F, both Systems 2 and 3 at the high NO source concentration produced a comparable or lower amount of NO₂ at a set dose of 40 ppm as the conventional NO delivery system at a lower NO source concentration. While not wishing to be bound by any particular theory, it is believed that the relatively low NO₂ values for Systems 2 and 3 at 40 ppm is a result of the FGF and NO-containing gases having similar velocities. As can be seen from Table 3 below, the velocity of the NO-containing gas was most similar to the FGF velocity at 40 ppm for the particular configurations tested as Systems 2 and 3.

**Table 3 - Velocity of NO-Containing Gas for Systems 2 and 3**

| | | NO Velocity (cm/sec) | | | | |
|---|---|---|---|---|---|---|
| | | NO Dose | | | | |
| | | 5 | 10 | 20 | 40 | 80 |
| FGF Velocity (cm/sec) | 3.67 | 0.4 | 0.9 | 1.8 | 3.6 | 7.3 |
| | 14.7 | 1.8 | 3.6 | 7.2 | 14.5 | 29.2 |
| | 58.79 | 7.2 | 14.4 | 28.9 | 58 | 117 |
| | 110.23 | 13.5 | 27 | 54.1 | 108.7 | 219.3 |
| | 220.46 | 27 | 54 | 108.3 | 217.5 | 438.6 |
| | 440.92 | 54 | 108.1 | 216.6 | 435 | 877.2 |

### EXAMPLE 2 - NO₂ GENERATION WITH HEATED SYSTEM

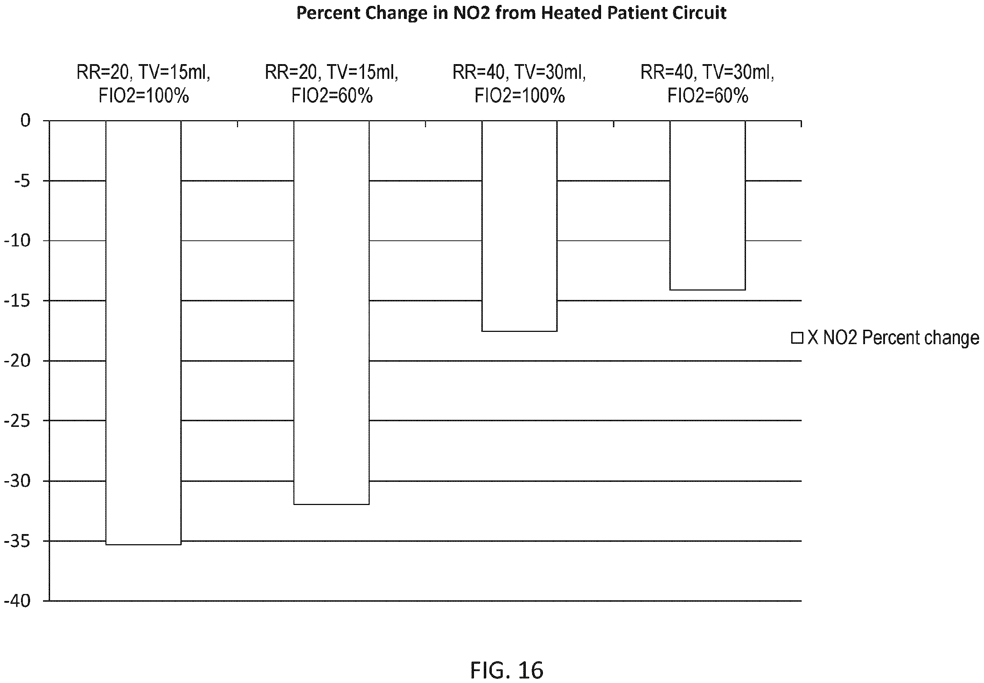

The NO delivery system used in System 2 of Example 1 was then used with a heated ventilator breathing circuit (e.g. about 38 °C). As can be seen from FIG. 16, heating the ventilator breathing circuit reduced the NO₂ levels under all conditions tested.

### EXAMPLE 3 - NO₂ MINIMIZATION USING GAS VELOCITY RATIO

The NO delivery system used in System 2 of Example 1 was modified to have various NO source concentrations and to provide various ratios of the FGF velocity to the NO-containing gas velocity. A plurality of gas sampling points was used for NO and NO₂ concentration measurements, which was averaged to account for any inhomogeneous distribution of the gases within the cross-section of the tube. The NO₂ concentration was measured at three different points downstream from the NO injection point T0: T1 (203 mm downstream from NO injection point), T2 (673 mm downstream from NO injection point) and T3 (2268 mm downstream from NO injection point). For the experiments described below, the region from T0 to T1 was considered to have a non-homogenous gas distribution and the region from T2 to T3 was considered to have a homogenous gas distribution. The NO₂ conversion rate was determined by subtracting the NO₂ contribution from the NO source cylinder from the measured NO₂ concentration, and dividing the net gain in NO₂ concentration by the residence time between sample points (volumetric flow rate divided by the volume of the segment).

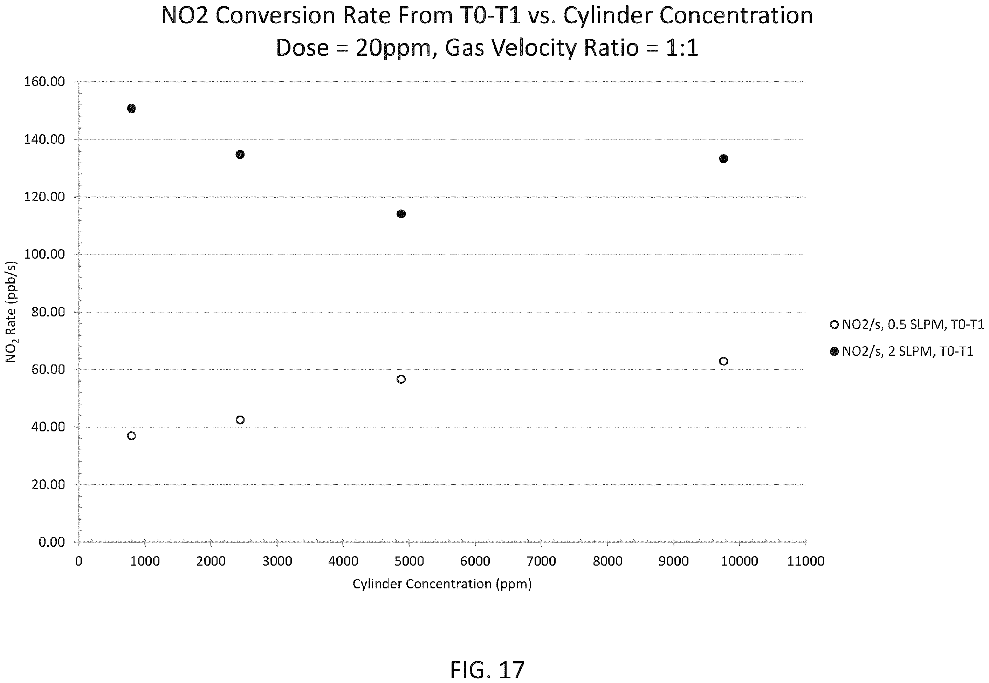

FIG. 17 shows the NO₂ generated in the initial T0-T1 region with various NO source cylinder concentrations ranging from 800 ppm to 9760 ppm with a gas velocity ratio (FGF : NO) of approximately 1:1. As can be seen from FIG. 17, by having a gas velocity ratio of approximately 1:1, the NO₂ generation rate is comparable between various cylinder concentrations at the same set dose (20 ppm) and the same FGF flow rate (0.5 or 2 SLPM).

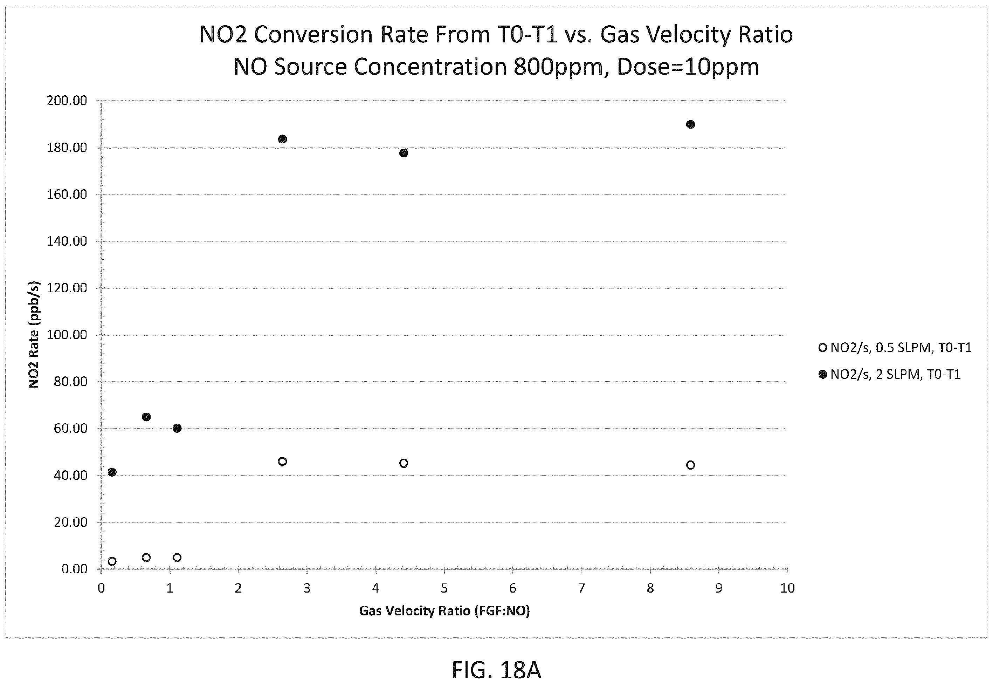

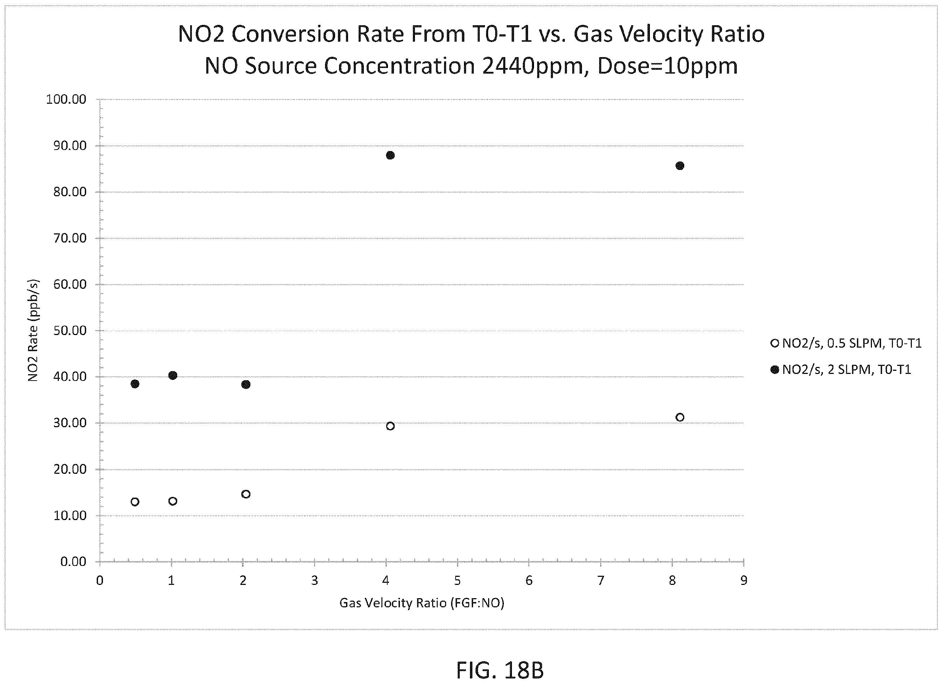

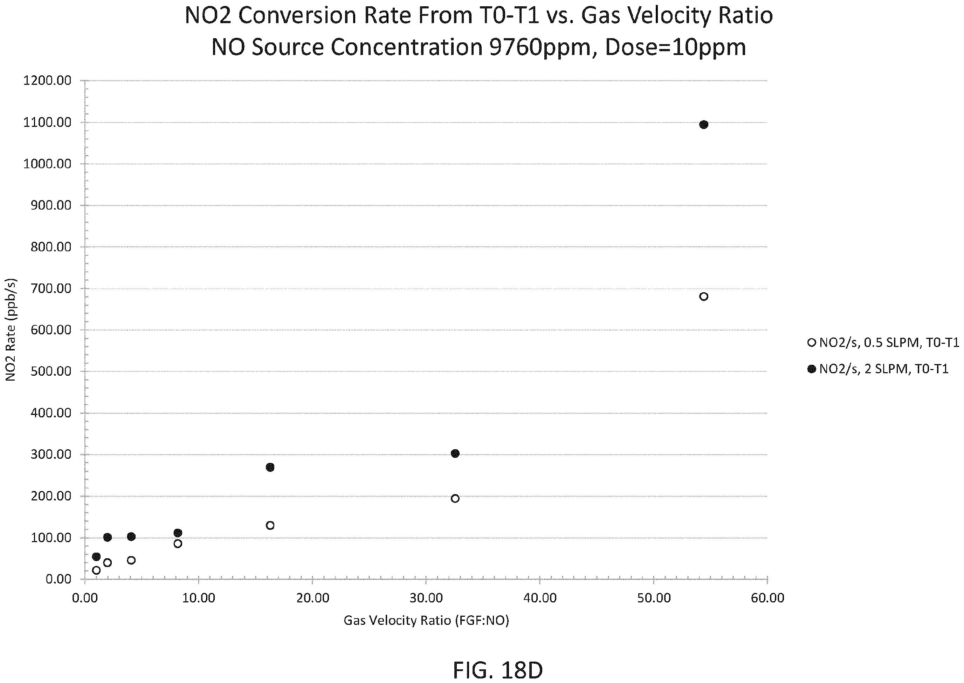

FIGS. 18A-D show the NO₂ generated in the initial T0-T1 region with various NO source cylinder concentrations ranging from 800 ppm to 9760 ppm with a varying gas velocity ratio (FGF:NO) and a set dose of 10 ppm NO. As can be seen from each of FIGS. 18A-D, gas velocity ratios below 2:1 provide a lower NO₂ generation rate than gas velocity ratios above 2:1, even when the NO source concentration, FGF flow rate and the NO set dose are the same.

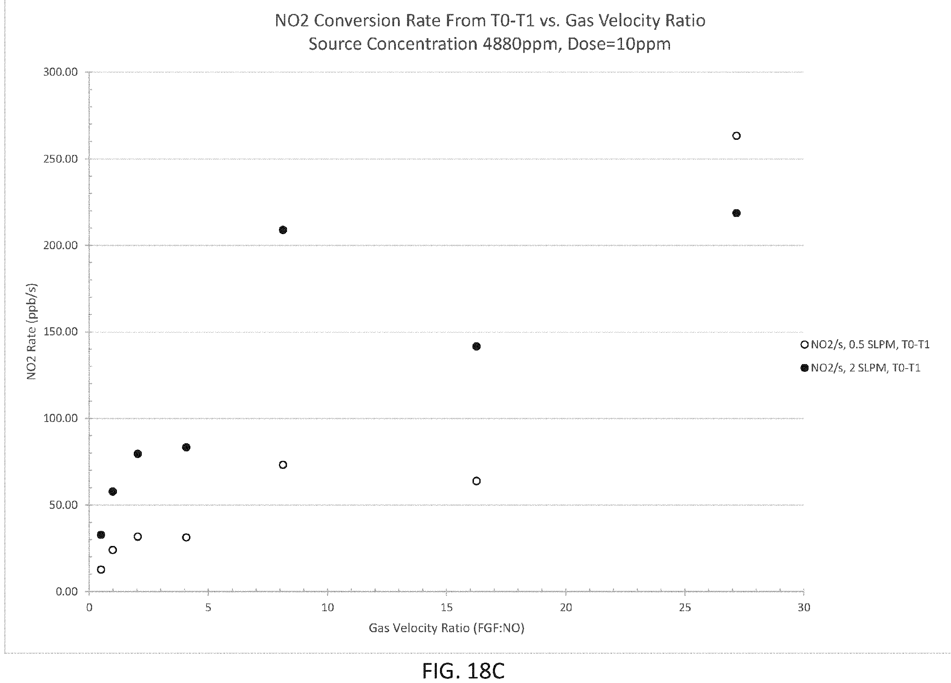

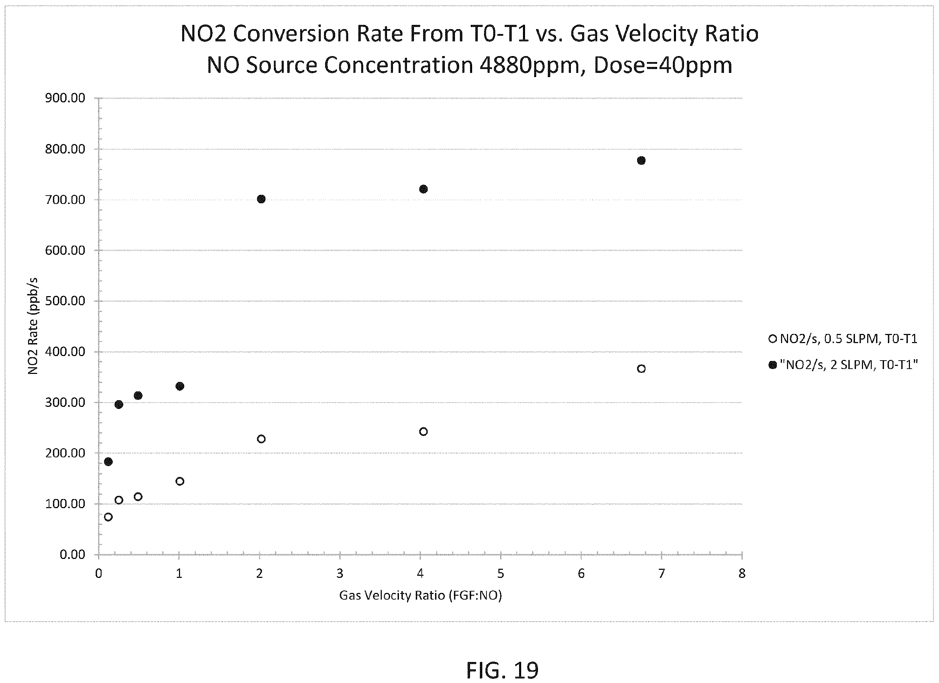

FIG. 19 shows the NO₂ generated in the initial T0-T1 region with a 4880 ppm NO source cylinder concentration and a set dose of 40 ppm, with a varying gas velocity ratio (FGF:NO). As can be seen by comparing FIG. 19 and FIG. 18C, the relationship between NO₂ generation rate and gas velocity ratio is also seen at other set dose concentrations.

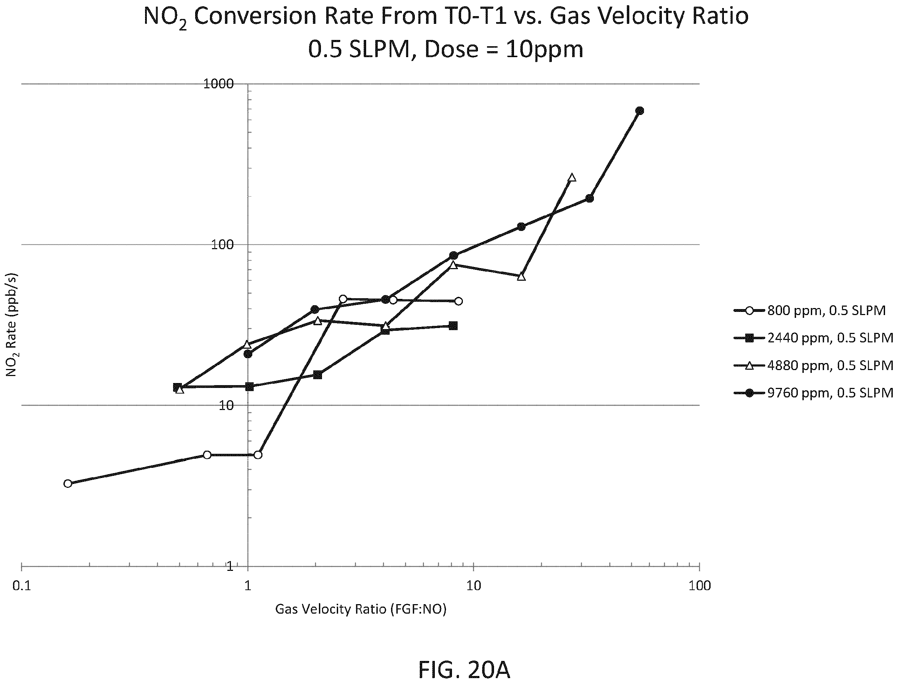

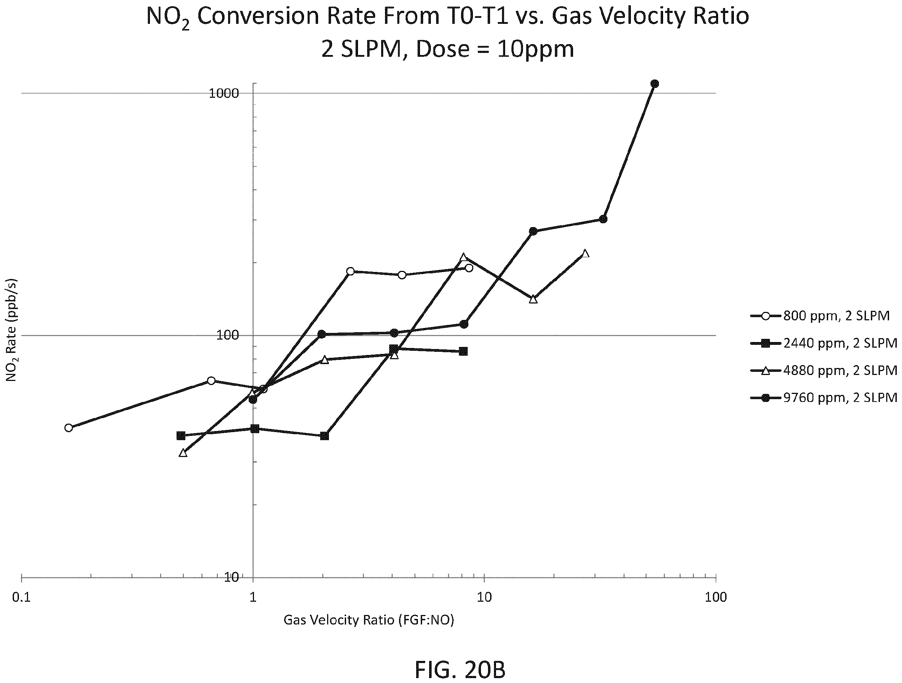

FIGS. 20A-B show the NO₂ generated in the initial T0-T1 region with various NO source cylinder concentrations ranging from 800 ppm to 9760 ppm with a varying gas velocity ratio (FGF:NO) and a set dose of 10 ppm NO. As can be seen from FIGS. 20A-B, gas velocity ratios below 2:1 provide a lower NO₂ generation rate than gas velocity ratios above 2:1, even when the NO source concentration, FGF flow rate and the NO set dose are the same. As FIGS. 20A-B are plotted on a logarithmic base 10 scale for both the x and y axes, this demonstrates that the instantaneous NO₂ generation is non-linear.

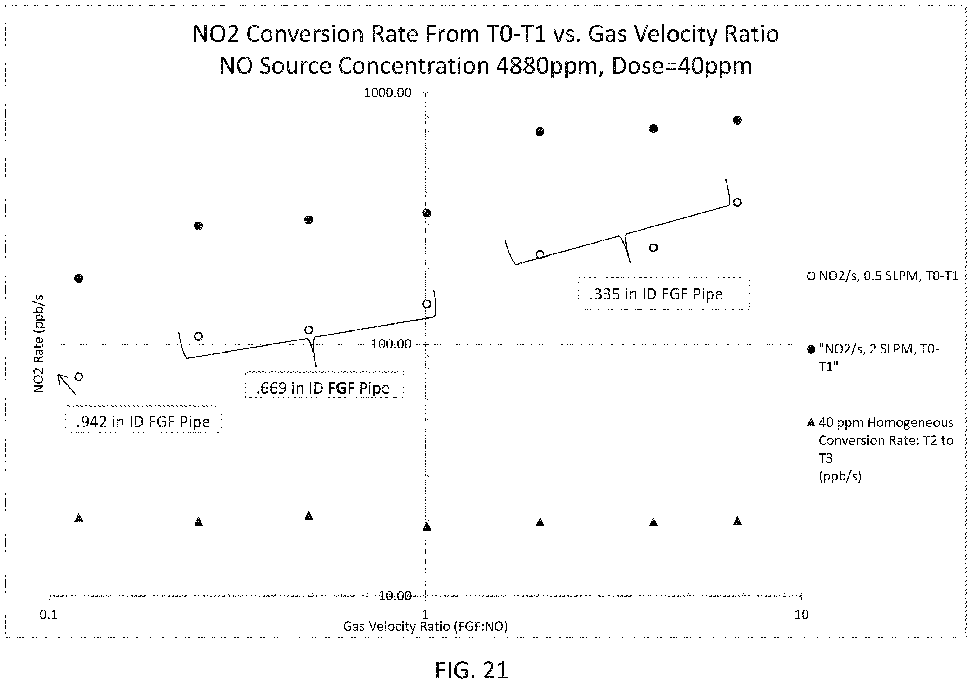

FIG. 21 shows the NO₂ generated in the initial T0-T1 region with a 4880 ppm NO source cylinder concentration and a set dose of 40 ppm, with a varying gas velocity ratio (FGF:NO). FIG. 21 also shows the average NO₂ generation rate from T2 to T3. As can be seen from FIG. 21, the NO₂ generation rate from T0-T1 is significantly higher than the NO₂ generation rate from T2 to T3. Also, the NO₂ generation rate from T2 to T3 (shown in triangles) does not vary with the gas velocity ratio, showing that a constant rate of NO₂ generation rate is achieved after the combined gas stream reaches a homogenous phase at T2. FIG. 21 further provides the size of the inner diameter of the FGF pipe for each configuration: 0.942 in, 0.669 in or 0.335 in. As can be seen, decreasing the FGF pipe diameter did not reduce NO₂ generation, but instead resulted in higher NO₂ generation rates. This is consistent with the observed phenomenon of NO₂ generation being minimized with lower FGF:NO velocity ratios, particularly those below 2:1.

### EXAMPLE 4 - NO₂GENERATION DURING CYCLING FLOW

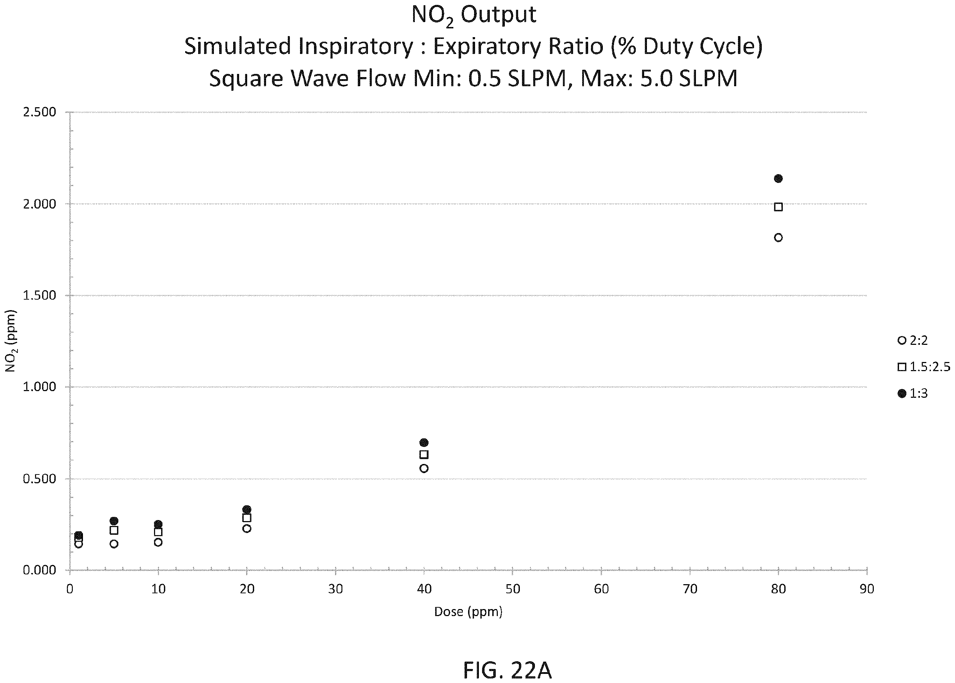

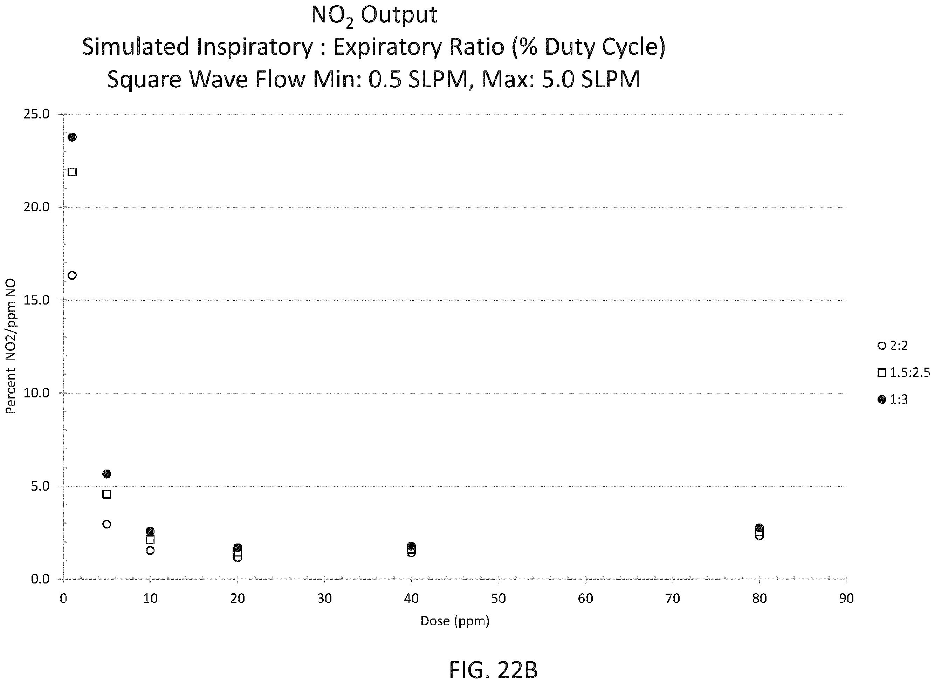

The NO delivery system of Example 3 was modified to simulate a ventilator with varying flow rates. A square wave flow with a minimum flow of 0.5 SLPM and a maximum flow of 5 SLPM was used, with a varying inspiratory to expiratory ratio (high to low flow ratio) ranging from 2:2 to 1:3. FIGS. 22A-B show the NO₂ generated in ppm and as a percentage of the set dose of NO. As can be seen from FIGS. 22A-B, the most NO₂ was generated with a higher expiratory (low flow) ratio. As can be seen from FIG. 22B, a high percentage of the NO was converted to NO₂ at the low set doses, with almost 25% of the NO being converted to NO₂ when the inspiratory : expiratory ratio was 1:3 and the NO set dose was 1 ppm.

### EXAMPLE 5: NO₂GENERATION SYSTEM COMPARISON

The NO₂ generation rate of a NO delivery system utilizing a suspended funnel (System 3 of Example 1) and the NO delivery system of Example 3 was compared from T0 to T1 at a set dose of 10 ppm NO and a cylinder concentration of 4880 ppm NO. The results of this comparison are shown in Table 4 below.

**Table 4 - NO₂ Generation for Systems 3 of Example 1 and Systems of Example 3**

| | | NO₂ Generation Rate (ppb/s) | | | |
|---|---|---|---|---|---|
| | | Suspended Funnel | Diffuser with Gas Velocity Ratio (FGF:NO) 2:1 | Diffuser with Gas Velocity Ratio (FGF:NO) 1:1 | Diffuser with Gas Velocity Ratio (FGF:NO) 0.5:1 |
| FGF Flow Rate (SLPM) | 0.5 | 32 | 32 | 24 | 13 |
| | 2 | 73 | 79 | 58 | 33 |

As can be seen from Table 4, the suspended funnel design performed comparably to a diffuser with a gas velocity ratio of about 2:1. However, diffusers with velocity ratios below 2:1 (1:1 or 0.5:1) provided lower NO₂ generation rates than the suspended funnel design.

## Claims

1. A device (100) for use in combining a first gas stream comprising molecular oxygen (O2) and a second gas stream comprising nitric oxide (NO) for delivery of a combined gas stream to a patient, **characterized in that** the diffusing of NO and O₂ occurs sufficiently rapidly that less than 1 ppm of NO₂ is delivered to the patient, wherein the second gas stream is provided by an NO source having an NO concentration of greater than 800 ppm to about 10,000 ppm.

2. The device (100) for use of claim 1, wherein the combined gas stream has an NO concentration of about 1 ppm to about 80 ppm.

3. The device (100) for use of claim 1 or 2, wherein the first gas is a breathable gas comprising molecular N₂ and molecular O₂, and the second gas comprises molecular NO and molecular N₂.

4. The device (100) for use of any one of claims 1-3, wherein the second gas stream initially enters the first gas stream at an angle in the range of about 60° to about 120°.

5. The device (100) for use of any one of claims 1-4, wherein the volumetric flow rate of the second gas is linearly proportional to the volumetric flow rate of the first gas.

6. The device (100) for use of any one of claims 1-5, wherein the first gas stream has a first velocity and the second gas stream has a second velocity, and the ratio of the first velocity to the second velocity is less than 2:1.

7. The device (100) for use of claim 6, wherein the ratio of the first velocity to the second velocity is less than or equal to about 1:1 and is provided when the first gas stream has a volumetric flow rate of less than 2 SLPM.

8. The device (100) for use of any one of claims 1-7, wherein the second gas stream enters the first gas stream at or near the central midpoint of the highest velocity of the first gas stream.

9. The device (100) for use of any one of claims 1-8, wherein the second gas stream is injected into the first gas stream as a plurality of pulses.

10. The device (100) for use of any one of claims 1-9, wherein the device comprises:
a body (110) comprising a wall (115) having a thickness, an outer surface, and an inner surface surrounding a hollow internal region (118);
a projection (195) extending from the inner surface of the body and into the hollow internal region; and
an injection channel (180) passing through the wall and projection to an injection port (185) such that the injection port injects the high concentration gas into the transverse gas stream at a distance from the inner surface of the body.

11. The device (100) for use of claim 10, wherein the hollow internal region (118) has a diameter, and a length of the projection (195) from the inner surface to the injection port outlet is in the range of about 30% to about 45% of the diameter of the hollow internal region.

12. The device (100) for use of claim 10 or 11, wherein the device comprises a plurality of injection ports (185).

13. The device (100) for use of any one of claims 1-12, wherein the device is integral to an injector module (605) comprising a flow sensor (615).

14. The device (100) for use of any one of claims 1-13, wherein the NO₂ generation is minimized by one or more of the location of injection of the second gas stream into the first gas stream, the first velocity, the second velocity, and/or the ratio of the first velocity to the second velocity.

## Patentansprüche

1. Gerät (100) zur Verwendung beim Kombinieren eines ersten Gasstroms, der molekularen Sauerstoff (O₂) umfasst, und eines zweiten Gasstroms, der Stickoxid (NO) umfasst, zur Abgabe eines kombinierten Gasstroms an einen Patienten, **dadurch gekennzeichnet, dass** die Diffusion von NO und O₂ ausreichend schnell erfolgt, dass weniger als 1 ppm NO₂ an den Patienten abgegeben wird, wobei der zweite Gasstrom durch eine NO-Quelle bereitgestellt ist, die eine NO-Konzentration von größer als 800 ppm bis etwa 10.000 ppm aufweist.

2. Gerät (100) zur Verwendung nach Anspruch 1, wobei der kombinierte Gasstrom eine NO-Konzentration von etwa 1 ppm bis etwa 80 ppm aufweist.

3. Gerät (100) zur Verwendung nach Anspruch 1 oder 2, wobei das erste Gas ein atembares Gas ist, das molekulares N₂ und molekulares O₂ umfasst, und das zweite Gas molekulares NO und molekulares N₂ umfasst.

4. Gerät (100) zur Verwendung nach einem der Ansprüche 1-3, wobei der zweite Gasstrom anfänglich in den ersten Gasstrom in einem Winkel in dem Bereich von etwa 60 ° bis etwa 120 ° eintritt.

5. Gerät (100) zur Verwendung nach einem der Ansprüche 1-4, wobei die Volumendurchflussrate des zweiten Gases linear proportional zu der Volumendurchflussrate des ersten Gases ist.

6. Gerät (100) zur Verwendung nach einem der Ansprüche 1-5, wobei der erste Gasstrom eine erste Geschwindigkeit aufweist und der zweite Gasstrom eine zweite Geschwindigkeit aufweist und das Verhältnis der ersten Geschwindigkeit zu der zweiten Geschwindigkeit weniger als 2:1 ist.

7. Gerät (100) zur Verwendung nach Anspruch 6, wobei das Verhältnis der ersten Geschwindigkeit zu der zweiten Geschwindigkeit weniger als oder gleich etwa 1:1 ist und bereitgestellt wird, wenn der erste Gasstrom eine Volumendurchflussrate von weniger als 2 SLPM aufweist.

8. Gerät (100) zur Verwendung nach einem der Ansprüche 1-7, wobei der zweite Gasstrom in den ersten Gasstrom an oder nahe dem zentralen Mittelpunkt der höchsten Geschwindigkeit des ersten Gasstroms eintritt.

9. Gerät (100) zur Verwendung nach einem der Ansprüche 1-8, wobei der zweite Gasstrom in den ersten Gasstrom als eine Vielzahl von Impulsen eingespritzt wird.

10. Gerät (100) zur Verwendung nach einem der Ansprüche 1-9, wobei das Gerät Folgendes umfasst:
einen Körper (110) umfassend eine Wand (115) mit einer Dicke, eine Außenfläche und eine Innenfläche, die eine hohle Innenregion (118) umgibt;
einen Vorsprung (195), der sich von der Innenfläche des Körpers in die hohle Innenregion erstreckt; und
einen Einspritzkanal (180), der durch die Wand verläuft und zu einem Einspritzanschluss (185) vorsteht, sodass der Einspritzanschluss das hochkonzentrierte Gas in den Quergasstrom in einem Abstand von der Innenfläche des Körpers einspritzt.

11. Gerät (100) zur Verwendung nach Anspruch 10, wobei die hohle Innenregion (118) einen Durchmesser aufweist und eine Länge des Vorsprungs (195) von der Innenfläche zu dem Einspritzanschlussauslass in dem Bereich von etwa 30 % bis etwa 45 % des Durchmessers der hohlen Innenregion ist.

12. Gerät (100) zur Verwendung nach Anspruch 10 oder 11, wobei das Gerät eine Vielzahl von Einspritzanschlüssen (185) umfasst.

13. Gerät (100) zur Verwendung nach einem der Ansprüche 1-12, wobei das Gerät einstückig mit einem Einspritzermodul (605) ist, das einen Durchflusssensor (615) umfasst.

14. Gerät (100) zur Verwendung nach einem der Ansprüche 1-13, wobei die NO₂-Erzeugung durch eines oder mehrere von der Stelle der Einspritzung des zweiten Gasstroms in den ersten Gasstrom, die erste Geschwindigkeit, die zweite Geschwindigkeit und/oder das Verhältnis der ersten Geschwindigkeit zu der zweiten Geschwindigkeit minimiert wird.

## Revendications

1. Dispositif (100) destiné à être utilisé pour combiner un premier flux de gaz comprenant de l'oxygène moléculaire (O2) et un second flux de gaz comprenant de l'oxyde nitrique (NO) pour délivrer un flux de gaz combiné à un patient, **caractérisé en ce que** la diffusion de NO et d'O₂ se produit suffisamment rapidement pour que moins de 1 ppm de NO₂ soit délivré au patient, dans lequel le second flux de gaz est fourni par une source de NO ayant une concentration de NO supérieure à 800 ppm à environ 10 000 ppm.

2. Dispositif (100) à utiliser selon la revendication 1, dans lequel le flux de gaz combiné a une concentration en NO d'environ 1 ppm à environ 80 ppm.

3. Dispositif (100) destiné à être utilisé selon la revendication 1 ou 2, dans lequel le premier gaz est un gaz respirable comprenant du N₂ moléculaire et du O₂ moléculaire, et le second gaz comprend du NO moléculaire et du N₂ moléculaire.

4. Dispositif (100) destiné à être utilisé selon l'une quelconque des revendications 1 à 3, dans lequel le second flux de gaz pénètre initialement dans le premier flux de gaz à un angle compris entre environ 60° et environ 120°.

5. Dispositif (100) destiné à être utilisé selon l'une quelconque des revendications 1 à 4, dans lequel le débit volumétrique du second gaz est linéairement proportionnel au débit volumétrique du premier gaz.

6. Dispositif (100) destiné à être utilisé selon l'une quelconque des revendications 1 à 5, dans lequel le premier flux de gaz a une première vitesse et le second flux de gaz a une seconde vitesse, et le rapport entre la première vitesse et la seconde vitesse est inférieur à 2:1.

7. Dispositif (100) destiné à être utilisé selon la revendication 6, dans lequel le rapport entre la première vitesse et la seconde vitesse est inférieur ou égal à environ 1:1 et est fourni lorsque le premier flux de gaz a un débit volumétrique inférieur à 2 SLPM.

8. Dispositif (100) destiné à être utilisé selon l'une quelconque des revendications 1 à 7, dans lequel le second flux de gaz pénètre dans le premier flux de gaz au niveau ou à proximité du point médian central de la vitesse la plus élevée du premier flux de gaz.

9. Dispositif (100) destiné à être utilisé selon l'une quelconque des revendications 1 à 8, dans lequel le second flux de gaz est injecté dans le premier flux de gaz sous la forme d'une pluralité d'impulsions.

10. Dispositif (100) destiné à être utilisé selon l'une quelconque des revendications 1 à 9, dans lequel le dispositif comprend :
un corps (110) comprenant une paroi (115) ayant une épaisseur, une surface extérieure et une surface intérieure entourant une région interne creuse (118) ;
une saillie (195) se prolongeant depuis la surface intérieure du corps et dans la région interne creuse ; et
un canal d'injection (180) traversant la paroi et faisant saillie vers un orifice d'injection (185) de sorte que l'orifice d'injection injecte le gaz à haute concentration dans le flux de gaz transversal à distance de la surface interne du corps.

11. Dispositif (100) destiné à être utilisé selon la revendication 10, dans lequel la région interne creuse (118) a un diamètre, et une longueur de la saillie (195) de la surface interne à la sortie de l'orifice d'injection est dans la plage d'environ 30 % à environ 45 % du diamètre de la région interne creuse.

12. Dispositif (100) destiné à être utilisé selon la revendication 10 ou 11, dans lequel le dispositif comprend une pluralité d'orifices d'injection (185).

13. Dispositif (100) destiné à être utilisé selon l'une quelconque des revendications 1 à 12, dans lequel le dispositif fait partie intégrante d'un module d'injecteur (605) comprenant un capteur de débit (615).

14. Dispositif (100) destiné à être utilisé selon l'une quelconque des revendications 1 à 13, dans lequel la génération de NO₂ est minimisée par un ou plusieurs éléments parmi l'emplacement d'injection du second flux de gaz dans le premier flux de gaz, la première vitesse, la seconde vitesse et/ou le rapport de la première vitesse à la seconde vitesse.
